(19) Europäisches Patentamt / European Patent Office / Office européen des brevets

(11) **EP 4 641 578 A1**

(12) **EUROPEAN PATENT APPLICATION**

(43) Date of publication:
29.10.2025  Bulletin 2025/44

(21) Application number: 24172640.5

(22) Date of filing: 26.04.2024

(51) International Patent Classification (IPC):
**G16H 20/10** (2018.01)    **G16H 70/40** (2018.01)

(52) Cooperative Patent Classification (CPC):
**G16H 20/10; G16H 70/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universität Basel**
**4001 Basel (CH)**

(72) Inventors:
• **BRAEM, Dominic**
  **8952 Schlieren (CH)**
• **KOCH, Gilbert**
  **88662 Überlingen (DE)**
• **PFISTER, Marc**
  **3652 Hilterfingen (CH)**

(74) Representative: **Bittner, Peter et al**
**Peter Bittner und Partner**
**Herrenwiesenweg 2**
**69207 Sandhausen (DE)**

(54) **COMPUTER SYSTEM AND METHOD FOR GENERATING A DATA DRIVEN INTERPRETABLE PHARMACOMETRICS PREDICTION MODEL TO PREDICT MEDICAL STATE PARAMETERS OF A PATIENT OF A SPECIAL POPULATION**

(57)    Computer system (100) and method for generating a data driven interpretable pharmacometrics prediction model (150) to predict at least one medical state parameter (MSP1) of a patient (201) of a special population. The system obtains a low-dimensional NODE (110) having an autonomous part and a non-autonomous part. The NODE comprises a first neural network (NN1) trained for approximating the autonomous part (AD), and a second neural network (NN2) trained for approximating the non-autonomous part (NAD). Training comprises fitting the NODE (110) to respective data (211) of respective special population members. Derivative data (AD', NAD') generated from the neural networks (NN1, NN2) is computed within minimum and maximum values of observed medical state parameter values (OPR) and time values (OTI). Outputs (O1 to OM) of predefined commonly accepted pharmacometric components (PC1 to PCN) are fitted to the derivative data using LASSO regression. A particular combination (C1) of best-fit pharmacometric components (PC1, PC2) is identified as the data driven interpretable pharmacometric prediction model (150).

FIG. 1

## Description

## Technical Field

The present invention relates to the field of pharmacometrics in general, and in particular, relates to methods, computer program products and systems for generating a data driven interpretable pharmacometrics prediction model to predict medical state parameters of a patient belonging to a special population.

## Background

In pharmacometrics (PMX), clinical data, such as drug concentration, biomarker measurements or clinical endpoints, is analyzed with quantitative methods and described with mechanistic mathematical-statistical models. A definition for clinical endpoints is given in section 1.1 of the EUnetHTA Guideline "Endpoints used for relative effectiveness assessment of pharmaceuticals: CLINICAL ENDPOINTS", Final version February 2013 (cf., https://www.eunethta.eu/wp-content/uploads/2018/01/Clinical-endpoints.pdf). Mathematical-statistical modeling in pharmacology, also called pharmacometrics (PMX), is an interdisciplinary field that applies ordinary differential equations to support decision-making in drug research, development, and regulatory review, as well as utilization of drugs, clinical care and practice. PMX modeling can be applied to all therapeutics, i.e. small molecule drugs and big molecule drugs such as peptides and biologics, gene therapy approaches, and clinical decision support systems.

Briefly, PMX modeling can be applied to characterize and predict drug concentrations, disease progression, and treatment effects. An essential concept in PMX modeling is non-linear mixed effects modeling or also called population analysis. Here it is assumed that patients with similar characteristics (e.g., suffering from a same disease) form a population and that the mathematical (structural) model, given by differential equations, is the same for every patient in this population. However, every patient has its own individual model parameters that follow given distributions and therefore, describe the inter-individual variability. Conventional PMX modeling approaches utilizing ordinary differential equations (ODEs) have the advantage that scientific concepts can be incorporated and the resulting model is explicitly formulated in terms of interpretable functions. However, PMX model development requires experienced PMX experts and can take 6-18 months depending on the complexity of described dynamics. Thereby, such dynamics can exist at multiple levels including, for example, organs (processing), immune system (reaction), pathophysiology, biomarker response, and chain of effects/responses. Further, PMX modeling is in some sense an iterative trial-and-error process since several models are developed and tested to characterize the given data. Basically, for every step in the model development, the given data is re-fitted again in the non-linear mixed effects framework. This leads to a large number of differential equations that must be solved. Therefore, the current PMX modeling approach is consequently computationally costly.

Particular challenges for PMX modeling pose special populations. For example, in a special population of pediatric patients, in particular in neonates and especially in pre-term neonates, strong maturation processes occur which must be characterized with sophisticated mathematical models. Typically, explicit time-dependencies describing maturation processes must be included in the PMX model, e.g., making the differential equations non-autonomous. Consequently, development of PMX models for neonatal populations is even more complex and time-consuming than for adult populations. Similar challenges exist for PMX modeling in relation to patients belonging to other special populations such as, a population consisting of elderly patients, a population consisting of pregnant and breastfeeding women, and a population consisting of patients with renal or hepatic impairment. It is to be noted that the term "special population" has a well-defined meaning in the medical field (cf. "Prescribing medicines for special populations" available at https://toolbox.eupati.eu/resources/prescribing-medicines-for-special-populations/ or "WHAT IS A SPECIAL POPULATION?" at https://www.nsca.com/certification/csps/what-is-a-special-population/).

Recently, a machine learning (ML) approach called neural ODEs (NODEs) was proposed to describe the dynamics of complex preclinical or clinical data. In contrast to conventional PMX approaches, NODEs automatically identify dynamics in complex datasets utilizing neural networks (NN). Thus, NODEs are highly efficient, i.e. they are capable of modeling complex dynamics within hours and days rather than months and years. However, because of the "black box" character of ML approaches, the resulting model does not allow for interpretation of the results which is inappropriate for medical personnel when using such a model in a diagnostic context to predict medical state parameters of individual patients.

## Summary

Hence, there is a need for a system and method to generate PMX models for patients of special populations (e.g., in pediatrics, particularly in newborns), such that the generated PMX models can handle complex physiological changes and time-dependent processes (e.g., maturation during the first years of life), and can be used by respective medically trained persons for reliable predictions of medical state parameters of said special population patients. This technical problem is solved by the features of a computer-implemented method, a respective computer program product and a computer system in accordance with the independent claims by initially training neural networks of a neural ordinary differential equation (NODE) to approximate the dynamics, i.e., the derivatives, of a particular medical state parameter in a special population. However, predictions of such neural networks cannot be explained and, therefore, can hardly be used in a meaningful way in a medical diagnostic or treatment context of human patients. Therefore, derivative data generated from the trained neural networks is then fit to output data of typical, generally accepted interpretable PMX functions. The best fit PMX functions reflect an interpretable model which provides results very similar to the non-interpretable predictions of the neural network and allows the use of such a purely data-driven generated model in the medical diagnostics/treatment context. That is, the tailored interpretable PMX model for a specific situation is generated fully automatically based on training data from a respective special population. Further, moving away from the prior art iterative trial-and-error process approach, computational cost is significantly reduced. This is achieved by applying NODEs and by transferring the entire deduction of the mechanism-based PMX model to a so-called derivative level.

A brief summary of non-linear mixed effects modeling is given in the following. Consider a population with n individuals. Every individual in the population has the same structural model given by the differential equation

$$\frac{d}{dt}x = f(x,t,\theta) \ , \quad x(0) = x^0 \tag{1}$$

where $f$ is the mechanism for the state $x$, and $\theta$ stands for the model parameters. The theoretical average patient in this population has the population parameters (typical values) $\theta_{pop}$. However, every individual has its own model parameters $\theta_i$ composed by $\theta_{pop}$, the individual covariates $Cov_i$ (e.g. weight, age, etc.) and the random effects $\eta_i$, characterizing unexplained variability. Hence, we have

$$\theta_i = h\big(\theta_{pop}, Cov_i, \eta_i\big) \quad with \ \ \eta_i \sim \mathcal{N}(0, \Omega^2)$$

where $\Omega$ is the standard deviation of the random effects and $h$ is a function describing the covariate model and the distribution of the individual model parameters for $i = 1, \ldots, n$. As example for $h$, let's consider the typical pharmacokinetics parameter *volume of distribution* V where we assume an effect of the individual weight $WT_t$ with a covariate power model, and log-normal distribution of the individual model parameter $V_i$. Then we have

$$V_i = h\big(V_{pop}, WT_i, \eta_{V,i}\big) = V_{pop} \cdot \left(\frac{WT_i}{70}\right)^{\beta} \cdot \exp(\eta_{V,i}) \quad with \ \ \eta_{V,i} \sim \mathcal{N}(0, \omega_V^2)$$

**Automation of modeling processes**

Major advances in the automation of modeling processes were made in automatically finding covariate effects on model parameters. Traditional methods like SCM (stepwise covariate modeling) test all possible combinations of covariate effects in a brute-force procedure, which results in extreme computational runtimes. These methods, however, are slowly but surely replaced by new methodologies like COSSAC (conditional sampling use for stepwise approach) or SAMBA (stochastic approximation for modeling building algorithm) that learn during covariate search. Such new methods can decrease computational time for covariate search. However, the situation for automatically finding the structural model Eq. (1) is different since the number of possible models is much larger and the decision-making for selecting a structural model is more complex compared to covariate modeling. Two prior art approaches are available:

- pyDarwin (cf. https://certara.github.io/pyDarwin/html/index.html) from the commercial company Certara, and
- Pharmpy (https://pharmpy.github.io/latest/index.html) from the University of Uppsala in collaboration with pharma company Hoffmann-La Roche, Basel, Switzerland.

Both approaches have in common that they are designed to find the best structural model by testing iteratively different standard PMX models. This requires first of all a toolbox of possible PMX models. However, the best PMX model is often not a standard model, but a tailored modeled including e.g. time-dependent terms. That is, prior knowledge about the dynamics to be modeled is required. Secondly, an algorithm finds the "best" PMX model either by machine learning approaches or by brute-force. Either way, solving differential equations multiple times is necessary.

## Neural ordinary differential equations

Neural networks are solely data-driven artificial intelligence (AI) / machine learning (ML) methods that learn to represent the behavior seen in respective training data. AI / ML gained new attraction in the PMX modeling world with the introduction of neural ordinary differential equations (NODE) (cf. Chen R, Rubanova Y, Bettencourt J, Duvenaud D. Neural Ordinary Differential Equations. arXiv:180607366. 2019). The idea is to substitute the right-hand-side of the differential equation Eq. (1) with neural networks. Hence, the derivatives are learned by the NNs. Therefore, the mechanism is no longer explicitly and iteratively modeled by hand, but is learned by the neural network. Thus, NODEs represent a data driven approach that does not require prior knowledge about the dynamics that has to be modeled. This turns out to be especially useful when complex dynamics have to be modeled. Two methods for applying NODEs to pharmacokinetics data were proposed in the prior art. The first method applies a common AI-based encoder/decoder approach (cf. Lu J, Deng K, Zhang X, Liu G, Guan Y. Neural-ODE for pharmacokinetics modeling and its advantage to alternative machine learning models in predicting new dosing regimens. iScience. 2021;24(7):102804). In the second method, so-called low-dimensional NODEs were developed (cf. Bram DS, Nahum U, Schropp J, Pfister M, Koch G. Low-dimensional neural ODEs and their application in pharmacokinetics. Journal of pharmacokinetics and pharmacodynamics. 2023). However, in both cases, the models are not interpretable (explainable) for the medically trained person.

Traditional PMX models are usually autonomous multi-dimensional ODE systems as they characterize pharmacological and physiological mechanisms. Since NODEs comprise neural networks and are purely data-driven, this multi-dimensionality is not necessary. Theoretically, every multi-dimensional ODE can be reduced to a non-autonomous onedimensional ODE (cf. Bram DS, Nahum U, Schropp J, Pfister M, Koch G. Low-dimensional neural ODEs and their application in pharmacokinetics. Journal of pharmacokinetics and pharmacodynamics. 2023). Hence, a general low-dimensional NODE structure, as disclosed in Bram et al., reads

$$\frac{d}{dt}x(t) = f_{NN}^1(x(t), \theta_{NN}^1) + x^0 \cdot f_{NN}^2(t, \theta_{NN}^2), \qquad x(0) = x^0 \qquad (2)$$

The neural network $f_{NN}^1(x(t), \theta_{NN}^1)$ is the autonomous part of the right-hand-side of the NODE describing the autonomous dynamics of at least one particular time-dependent medical state parameter x(t) with respect to the current x(t), i.e., the mechanism of the medical state parameter without additional specific behavior in the given special population. The neural network $f_{NN}^2(t, \theta_{NN}^2)$ is the non-autonomous part of the right-hand-side of the NODE describing the non-autonomous dynamics of at least one particular time-dependent medical state parameter x(t) with respect to the current time $t$, e.g., due to specific behavior in the given special population (cf., Riesbeck J., Systems of linear nonautonomous differential equations, Centre for Mathematical Sciences Lund University, 2020 ). For example, this can be maturation effects in a neonatal special population. Thereby, the measurement level is described by the state parameter x(t). The derivative level is described by the neural networks $f_{NN}^1(x(t), \theta_{NN}^1)$ and $f_{NN}^2(t, \theta_{NN}^2)$.

The additive structure of Eq. (2) does not hamper the flexibility of the NODE for PMX modeling. For example, the NODE structure Eq. (2) is suitable for fitting highly non-linear data (e.g., serum creatinine data from very pre-term neonates with pronounced gestational age dependent maturation processes, and even pharmacodynamics data of prothrombin complex activity).

A barrier to the application of such neural networks for practical medical application is the "black-box" behavior of neural networks. This disadvantage is overcome with the herein disclosed approach of an automated back-transformation of a learned NODE model into interpretable combinations of predefined commonly accepted pharmacometric components; e.g., a mechanistic or semi-mechanistic pharmacometric model that accounts for pharmacokinetic, pharmacodynamic, pharmacological, (patho-)physiological, biological, and/or clinical principles or processes.

In more detail, in one embodiment, a computer-implemented method is provided for generating a data driven interpretable prediction model to predict medical state parameters of a patient of a special population. For example, such medical state parameters include, but are not limited to, changes of body weight, changes of the electrolyte serum sodium, and changes of serum creatinine (a parameter for kidney function). The method starts by obtaining a low-dimensional neural ordinary differential equation (NODE). For example, the NODE may be used in a non-linear mixed effects (NLME) framework, or a pooled data fitting approach may be used. In the NLME framework, subjects are modeled simultaneously through the inclusion of random effects of subjects for kinetic parameters that are assumed to vary between subjects. Thereby, training the neural networks comprises fitting the NODE to respective data of said patients of a special population. One of the strengths of NODEs is that they can fit pharmacodynamic data (e.g., biomarkers such as Serum Creatinine). This is particularly relevant in the case of special population patients where complex and time-dependent effects play an important role.

The neural networks can be relatively small with a number of neurons in the order of 5 to 20 neurons per neural network. When fitting the NODE to the special population data, the initial values of the neural networks are set automatically.

Then, derivative data generated from the neural networks is computed within minimum and maximum values of observed medical state parameter values and time values. Since the neural networks in NODEs characterize the right-hand side of the differential equation (2), so-called derivative vs. state / time dependencies (or plots) can be used to assess the mechanism learned by the NODE.

The back-transformation of the NODE into an interpretable mechanistic pharmacometric model is then achieved by fitting the outputs of combinations of predefined commonly accepted pharmacometric components to the derivative data using a LASSO regression. The LASSO regression is based on a generalized linear model with L1 penalty. In other words, the LASSO regression uses L1 penalization of the weight parameters in the linear combination of the pharmacometric components, and sets the weight parameters of unnecessary pharmacometric components to zero. Commonly accepted pharmacometric components are known by a person of skill in the art of pharmacometrics and can be retrieved from. For example, the commonly applied pharmacometric components can be of any of the following types: linear function, exponential functions, sigmoidal functions, power functions, and hyperbolic functions as described for example on the model library websites of the PMX software Monolix (cf. https://monolix.lixoft.com/demo-projects/libraries-of-mo dels and https://monolix.lixoft.com/statistical-model/individual-model/covariate). A person skilled in the art may also use other component types if appropriate. Advantageously, the selection of the predefined commonly accepted pharmacometric components is received prior to fitting the outputs to the derivative data, wherein the selection comprises components having a visual similarity with corresponding derivative-versus-state plots of said derivative data. For example, the combinations of predefined commonly accepted pharmacometric components can be selected by a respectively trained neural network.

A particular combination of best-fit pharmacometric components is then identified as the data driven interpretable prediction model (with explicit deterministic functions) which best describes the learned functions of the first and second neural networks. It is to be noted that the herein disclosed computer-implemented method is executed by a computer system fully automatically and does not require any interaction with a human. It is to be noted that the deduced mechanism-based model can be close to the structure that would have been developed by a human PMX modeler. But this is not necessarily the case due to the low-dimensional nature of the applied NODE. As an example, consider a mechanistic TMDD model, as for example discussed by Koch G, Jusko WJ, Schropp J. in "Target mediated drug disposition with drug-drug interaction, Part II: competitive and uncompetitive cases." (Journal of pharmacokinetics and pharmacodynamics. 2017;44(1):27-42) which is in its original form a three-dimensional system. Obviously, the low-dimensional NODE approach will never reproduce the exact same structure of the TMDD model. However, it produces a non-autonomous structure which is at least close the original TMDD model behavior.

To summarize, the above proposed PMX modeling automation workflow leverages the learned knowledge from a trained low-dimensional NODE on a given dataset to deduce a mechanism-based PMX model. For that, the dynamics learned by the NODE is transformed (translated) back to an interpretable mechanism-based PMX model, meaning that no longer neural networks will appear in the right-hand side of the structural model, but a combination of explicit PMX components.

### Covariate search

In one embodiment, the method further comprises steps to find covariate effect models in the deduced mechanism-based PMX model.

For this purpose, the identified particular combination of pharmacometric components is fitted to derivative data of individual special population patients in the non-linear mixed effects modeling framework to determine inter-individual variability in the computed derivative data in form of random effects on model parameters in the combination of best-fit pharmacometric components. Then, the outputs of predefined covariate components are fitted to the random effects using a LASSO regression. A particular combination of best-fit covariate components is then identified as data driven interpretable covariate model which best describes the influence of covariates on individual dynamics in the special population. Finally, the interpretable covariate model (180) is merged into the interpretable pharmacometric prediction model (150).

### Medical state prediction of an individual patient

For making a medical state parameter prediction of an individual patient, the interpretable pharmacometrics prediction model (150) is implemented in a PMX software.

In a first implementation, one or more input parameters for the generated interpretable pharmacometric prediction model are obtained from a data source which provides such input parameters that relate to a particular patient with characteristics of the special population on which the NODE has been trained. For example, the data source may be a covariate record of the particular patient which includes the relevant covariate measures of said patient.

In a second implementation, one or more measurements of the medical state parameter of the particular patient with characteristics of the special population are obtained (e.g., from said patient record or directly from respective medical sensors). An Empirical Bayesian Estimation (EBE) step can be utilized to estimate individual model parameters given the previously estimated parameter distribution in the population and the previously observed medical state parameters for one individual patient (cf. https://monolix.lixoft.com/tasks/ebes).

The interpretable pharmacometric prediction model is then applied to said one or more inputs and/or said individual model parameters. Thereby, both implementations may also be used in combination to finally provide the at least one medical state parameter of said particular patient.

In one embodiment, a computer program product is provided for generating a data driven interpretable prediction model to predict medical state parameters of a patient of a special population. The computer program product comprises computer readable instructions that, when loaded into a memory of a computing device and executed by at least one processor of the computing device, cause the computing device to execute the herein disclosed computer-implemented method.

In one embodiment, a computer system is provided for generating a data driven interpretable prediction model to predict medical state parameters of a patient of a special population. The computer system has a memory to store computer readable instructions defining functional modules of the system, and has at least one processor which can process the computer readable instructions to instantiate such functional modules at runtime. The functional modules are thereby adapted to perform the steps of the computer-implemented method when being executed at runtime.

Further aspects of the invention will be realized and attained by means of the elements and combinations particularly depicted in the appended claims. It is to be understood that both, the foregoing general description and the following detailed description are exemplary and explanatory only and are not restrictive of the invention as described.

**Brief Description of the Drawings**

FIG. 1 shows a simplified diagram of an embodiment of a computer system for generating a data driven interpretable pharmacometrics prediction model to predict at least one medical state parameter of a patient of a special population;

FIG. 2A is a simplified flow chart illustrating a computer-implemented method for generating said data driven interpretable pharmacometrics prediction model according to an embodiment;

FIG. 2B illustrates optional sub-steps of the computer implemented method for adding an interpretable covariate model;

FIG. 2C illustrates optional sub-steps of the computer implemented method for predicting a medical state parameter of a particular patient;

FIG. 3 is a schematic overview illustrating the substitution of the right-hand-side of a differential equation with a neural network when using NODEs in PMX modeling;

FIG. 4A shows illustrative examples of the measurement level and derivative level based on a one-compartment pharmacokinetics model;

FIG. 4B illustrates an example of a derivative-vs.-time plot for derivative data generated from the non-autonomous part of a NODE;

FIG. 5 provides a schematic overview of the herein disclosed PMX modeling automation workflow including covariate search;

FIGs. 6A, 6B illustrate a derivative-versus-age plot example of fitted NNs in NODE with typical and individual estimated parameters, and a corresponding boxplot;

FIGs. 6C, 6D illustrate a derivative-versus-time plot of fitted NNs in NODE with typical and individual estimated parameters, and a corresponding boxplot;

FIG. 7A illustrates a serum creatinine concentration example with dynamics learned by the neural networks of NODE and interpretable PMX functions derived from the learned dynamics;

FIG. 7B illustrates covariate effects observed in the serum creatinine concentration example;

FIG. 7C is a simulation plot showing simulations of serum creatinine concentrations for various gestational ages with corresponding median birth weights;

FIG. 8A to 8C illustrate the generation of a neonatal weight PMX model according to an embodiment;

FIG. 9A to 9C illustrated the generation of a neonatal sodium PMX model according to an embodiment; and

FIG. 10 is a diagram that shows an example of a generic computer device and a generic mobile computer device, which may be used with the techniques described here.

**Detailed Description**

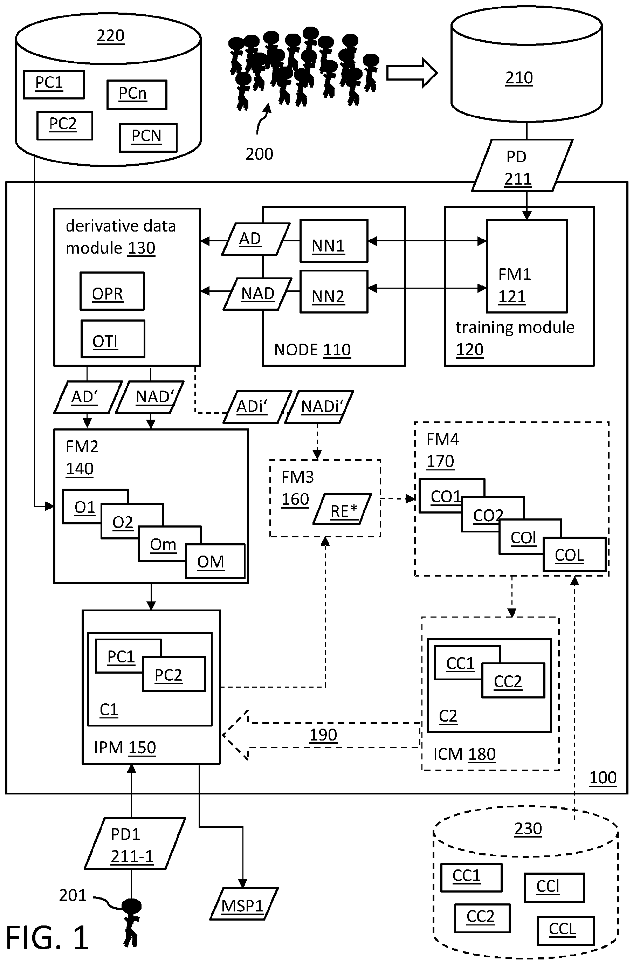

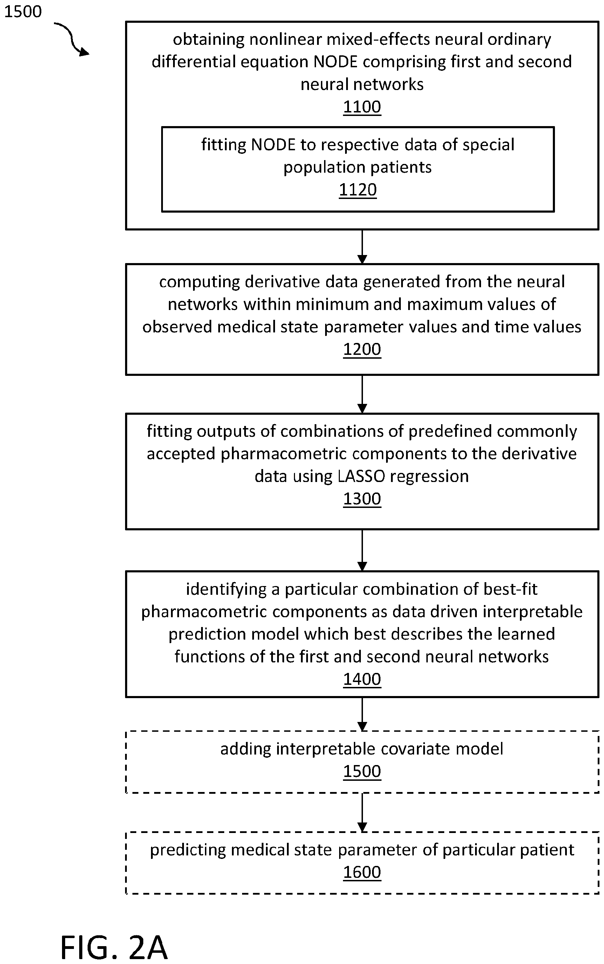

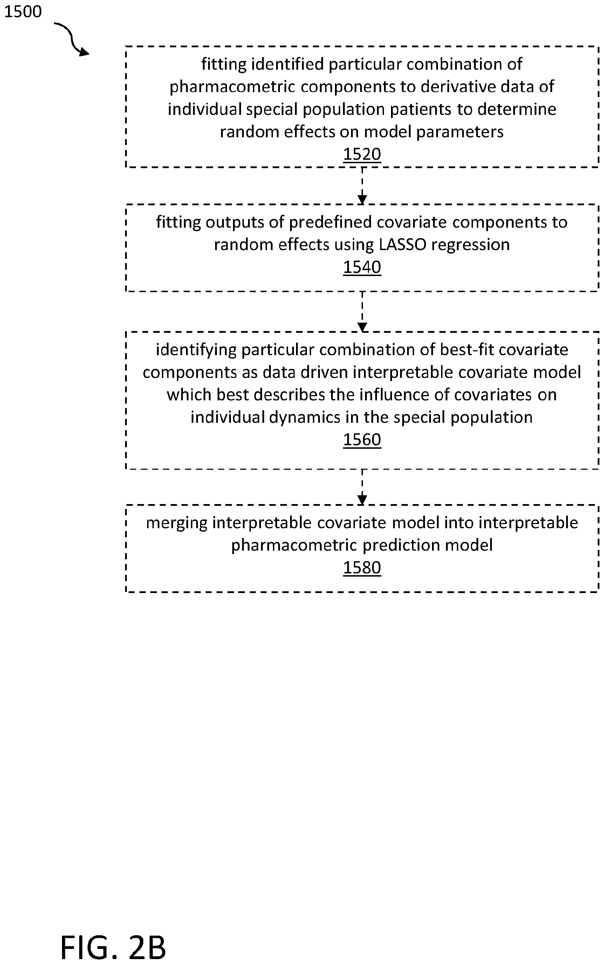

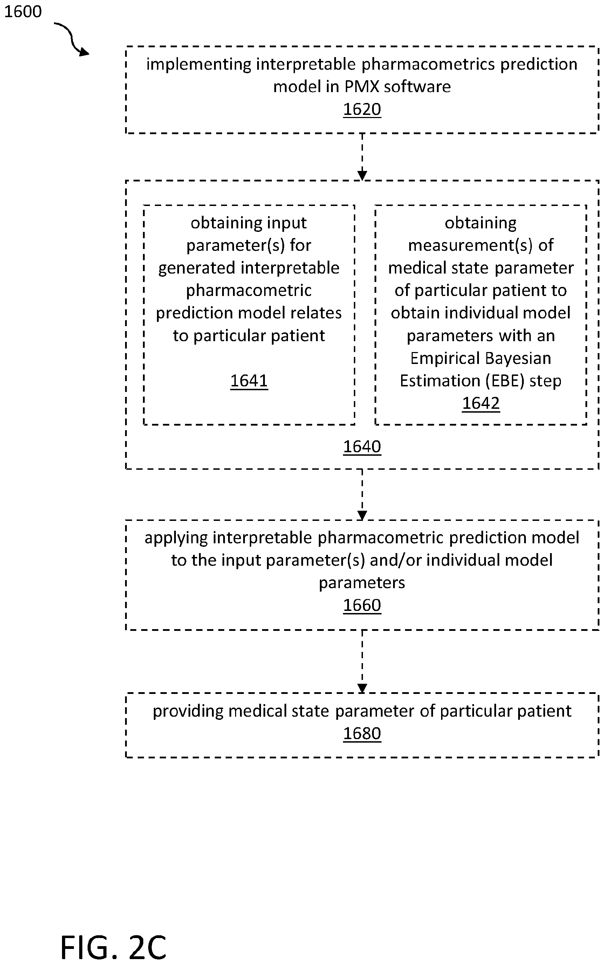

FIG. 1 shows a simplified diagram of an embodiment of a computer system 100 for generating a data driven interpretable pharmacometrics prediction model 150 to predict at least one medical state parameter MSP1 of a patient 201 of a special population. FIGs. 2A to 2C illustrate simplified flow charts of a computer-implemented method 1000 for generating said data driven interpretable pharmacometrics prediction model according to various embodiments. Thereby, the method steps can be executed by respective modules of system 100 which are configured accordingly. Therefore, the following description of FIG. 1 describes system 100 in the context of method 1000 and also refers to the refence numbers of FIGs. 2A to 2C.

System 100 has one or more interfaces (not shown) which are adapted to communicatively couple the system with external components such as data sources 210, 220, 230 and to allow user interaction (e.g., with user 210). It is to be noted that the data sources 210, 220, 230 may also be implemented as integrated components with system 100. The system includes a neural ordinary differential equation 110 (NODE) which may be obtained 1100, for example, from a respective library with mathematical functions via a respective interface.

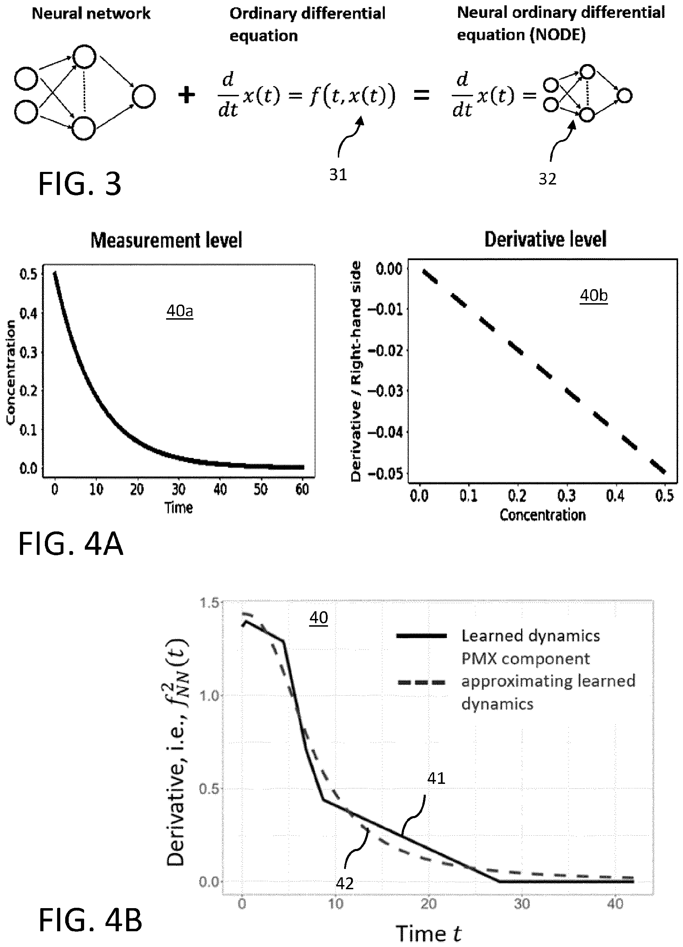

Turning briefly to FIG. 3, the idea when using NODEs in PMX modeling is to substitute the right-hand-side 31 of the differential equation Eq. (1) with a neural network 32. This allows to learn a mechanism from data and avoids explicit modeling of the mechanism by hand. In FIG. 3, a simplified notation of Eq. (1) is used where the model parameters $\theta$ are omitted.

NODE 110 is advantageously used in a non-linear mixed effects modeling framework, wherein the right-hand side of the NODE (cf., Eq. (2)) has an autonomous part and a nonautonomous part.

The autonomous part describes the autonomous dynamics AD of at least one particular time-dependent medical state parameter of a special population 200 of patients. The term special population is a well-known term used in the medical field for any one of the following populations: a population consisting of pediatric patients, a population consisting of elderly patients, a population consisting of pregnant and breastfeeding women, and a population consisting of patients with renal or hepatic impairment. In general, patients can be seen as the members of the mathematical sets that represent the respective special populations. Special populations typically show very fast change processes in the medical states of the respective patients. In NODE 110, a first neural network NN1 (cf., $f_{NN}^1$ in Eq. (2)) is trained for approximating this autonomous part. The training of the neural network NN1 is performed by a training module 120 using a first fitting module 121 adapted to fit 1120 the NODE 110 to respective data PD 211 of said special population patients 200. Such patient data PD 211 can be retrieved from a corresponding data source 210 which is used to store patient records of the special population 200. That is, the neural network NN1 can be trained with data from patients which share medical state properties that are characteristics of said special population. For example, a low-dimensional NODE of the form

$$\frac{dx}{dt} = f_{NN}^x(x, \theta_x) + x_0 \cdot f_{NN}^t(t, \theta_t), \qquad x(0) = x_0$$

may be fitted with the MonolixSuite software (available at https://www.simulations-plus.com/software/monolix/) to data of the special population (e.g., neonatal/pediatric data) and population parameters $\theta_x$ and $\theta_t$ can be estimated.

The non-autonomous part describes the non-autonomous dynamics NAD of at least one particular time-dependent medical state parameter of the special population patients 200. In NODE 110, a second neural network NN2 (cf., $f_{NN}^2$ in Eq. (2)) is trained for approximating this non-autonomous part. The training of the neural network NN2 is also performed by training module 120 using said first fitting module 121 for fitting 1120 the NODE 110 to respective data PD 211 of said special population patients 200. In other words, the NODE Eq. (2) learns the mechanism underlying the training data 211 in order to determine a structural model. Hence, the learned mechanism is represented by the two neural networks in Eq. (2). This requires solving the differential equation on the measurement level only once.

The approximations AD and NAD are then provided to a derivative data module 130 which computes 1200 derivative data AD', NAD' generated from the neural networks NN1, NN2 within minimum and maximum values of observed medical state parameter values OPR and time values OTI. The derivative AD' from the first neural network NN1 can be illustrated as a derivative vs. state plot. The derivative NAD' from the second neutral network NN2 can be illustrated as a derivative vs. time plot. For example, with regard to the neural network $f_{NN}^1(x, \theta_{NN,pop}^1)$ that describes one part of the derivative, i.e., the right-hand side of the NODE Eq. (2), $x_{min}$ is the lower bound and $x_{max}$ is the upper bound for state $x$, hence, $x \in [x_{min}, x_{max}]$. The interval $[x_{min}, x_{max}]$ corresponds to the parameter value range OPR of observed medical state parameter values within the minimum value $x_{min}$ and the maximum value $x_{max}$. The derivative data AD' at the "derivative level" is generated from the neural network $f_{NN}^1(x, \hat{\theta}_{NN,pop}^1)$, where $\hat{\theta}_{NN,pop}^1$ are the estimated population parameters. The derivative data NAD' at the "derivative level" is generated from the neural network

$$f_{NN}^2(t, \theta_{NN}^2) = f_{NN}^t(t, \theta_t)$$ with $t \in [t_{min}, t_{max}]$ wherein $t_{min}, t_{max}$ correspond to minimum and maximum of observed time values OTI.

FIG. 4A shows a simple illustrative example of the measurement level and derivative level based on a one-compartment pharmacokinetics (PK) model. A one-compartment PK model is a mono-exponential function for a drug concentration C(t) (shown in panel 40a) given by $\frac{d}{dt} C = -k_{el} \cdot C, \ \ C(0) = \frac{d}{V}$, where d is the administered dose, $k_{el}$ the elimination rate and V the volume of distribution. This is the measurement level where drug concentration measurements are available. In panel 40b the derivative level of the one-compartment PK model is shown, which is the right-hand side of the equation, i.e., $-k_{el} \cdot C$. Hence on the derivative level, the derivative vs. state behavior can be investigated. It is to be noted that this example poses the simplest case and is just for illustration purposes.

FIG. 4B illustrates an example of a derivative vs. time plot 40 for derivative data generated from $f_{NN}^2$. More examples are discussed in the context of specific application embodiments further down. In plot 40, the learned non-autonomous dynamics is illustrated by solid line 41. The dashed line 42 describes a potential PMX component g(t) that approximates the learned mechanism. On the derivative level no solving of the differential equation is necessary.

This potential PMX component is identified 1400 by a second fitting module FM2 140 of system 100 based on the derivative data. FM2 140 is adapted to fit 1300 outputs O1 to OM of predefined commonly accepted pharmacometric components PC1 to PCN to the derivative data AD', NAD' using a LASSO regression. Such commonly accepted pharmacometric components suitable for respective special populations are known in the art. The pharmacometric components are functions that are used in conventional PMX modeling. They can be for example sigmoidal terms, exponential terms, linear terms etc. These PMX components are basic elements that are combined to deduce the mechanism-based PMX model IPM 150 from the learned mechanism of the NODE Eq. (2). For example, the outputs of the pharmacometric components can be generated by using so-called "R Software Tools" known in the art. Let $g_i^x(x, \theta_i)$ and $g_i^t(t, \theta_i)$ be typical PMX functions (linear function, exponential functions with different exponent-parameters, sigmoidal functions, etc.) with a specific parameter set $\theta_i$ for this type of function. Using R, output data $dx_i^x = g_i^x(x, \theta_i)$ with $x \in [x_{min}, x_{max}]$ and $dx_i^t = g_i^t(t, \theta_i)$ with $t \in [t_{min}, t_{max}]$ can be generated. In one implementation, the generation of outputs O1 to OM can be an integrated function of FM2 140. In another implementation, the generation of outputs may occur in a separate module which provides the outputs to FM2 140.

In the example, data source 220 provides a pool of such commonly accepted pharmacometric components PC1 to PCN to system 100. The LASSO regression uses L1 penalization of the weight parameters in the linear combination of the pharmacometric components, and sets the weight parameters of unnecessary pharmacometric components to zero. FM2 140 can use the respective component outputs O1 to OM and perform the fitting 1300. A particular combination C1 of best-fit pharmacometric components PC1, PC2 is then identified 1400 as the data driven interpretable pharmacometric prediction model 150 which best describes the learned functions of the first and second neural networks NN1, NN2. In more detail, when applying the LASSO regression to obtain the PMX components that best fit the generated derivative data, LASSO selects a linear combination of the PMX components out of $g^1, \dots, g^n$ that describe best the behavior of $f_{NN}^1(x, \hat{\theta}_{NN,pop}^1)$. This is obtained by a L1 regularization of the weight parameters in the linear combination of the PMX components, consequently setting the weight parameters of PMX components that are not necessary to zero. The result of this minimization process is the combination of different PMX components representing the deduced mechanism-based PMX model. For example, the result may be:

$$\frac{d}{dt} x = g(t, x) = g^1(x) + g^2(x) + g^3(t) + g^4(t), \ \ x(0) = x^0$$

The software R may also be used for fitting the output data O1 to OM to the derivative data AD' and NAD', respectively, with the LASSO-regression. Functions $g_i^x(x, \theta_i)$ and $g_i^t(t, \theta_i)$ related to output data that are not excluded by the LASSO-regression are selected as relevant mechanistic functions.

The identified combination C1 describes the dynamics learned by said neural networks and can therefore be seen as a purely data driven model which provides, when applied by a medically trained person, a level of transparency of the model which supports a reliable diagnosis of the medical state of an individual patient. In other words, with this interpretable mechanism-based PMX model 150, neural networks no longer appear in the right-hand side of the structural model, but a combination of explicit PMX components PC1, PC2.

Moving from the measurement level to the derivative level, and applying the machine learning method LASSO regression to automatically deduce the mechanism-based PMX model from the learned mechanism of the NODE Eq. (2) by combining predefined PMX components that describe the dynamics of the learned mechanism given by the two neural networks, results in the mechanism-based PMX model 150 without a need for solving any differential equation.

FIG. 1 further illustrates an optional embodiment of system 100 further including optional modules (illustrated by dashed frames) for adding 1500 an interpretable covariate model ICM 180 which can enhance the generated interpretable PMX model IPM 150 such that an improved prediction accuracy is achieved.

In this optional embodiment, a third fitting module FM3 160 is adapted to fit 1520 the identified particular combination C1 of pharmacometric components to derivative data ADi', NADi' of individual special population patients in the non-linear mixed effects modeling framework to determine inter-individual variability in the computed derivative data in form of random effects on model parameters RE* in the combination C1 of best-fit pharmacometric components PC1, PC2. An example of such a model parameter is the slope of derivative curves. For illustration, only the neural network $f^1_{NN}\left(x, \theta^1_{NN,i}\right)$ is considered. Let $x_{min}$ be the lower bound and $x_{max}$ be the upper bound for the state $x$, hence, $x \in [x_{min}, x_{max}]$. Then, the individual derivative data is generated from the neural network $f^1_{NN}\left(x, \hat{\theta}^1_{NN,i}\right)$ where $\hat{\theta}^1_{NN,i}$ are the estimated individual parameters. FM3 the fits $g^1\left(x, \theta^{g1}_i\right) + g^2\left(x, \theta^{g2}_i\right)$ from above to the individual derivative from $f^1_{NN}\left(x, \theta^1_{NN,i}\right)$ and allows inter-individual variability (random effects) on the PMX component parameters for $g^1\left(x, \theta^{g1}_i\right) + g^2\left(x, \theta^{g2}_i\right)$.

A fourth fitting module FM4 170 is adapted to fit 1540 outputs CO1 to COL of predefined covariate components CC1 to CCL to the random effects RE* using a LASSO regression. In step 1540, LASSO selects a linear combination of covariate components, i.e., covariates and transformations of covariates (e.g., log-transformation), that describe best the inter-individual variability. It is to be noted that, in the LASSO terminology, sometimes the term "covariates" describes the features. However, in the medical terminology applied herein, "covariates" are the individual patient characteristics.

Similarly, as for the pharmacometric components, predefined and commonly accepted covariate components are known in the art and may be provided in a respective library 230. The outputs CO1 to COL may be generated by a function of FM4 170, or they may be generated by a separate module which provides the outputs to FM4. Further, FM4 170 identifies 1560 a particular combination C2 of best-fit covariate components CO1, CO2 as data driven interpretable covariate model 180 which best describes the influence of covariates on individual dynamics in the special population. This covariate search is still performed at the derivative level and applies again the LASSO method again to find appropriate covariate effect models in the deduced mechanism-based PMX model IPM 150. Again, no solving of differential equations is necessary when combining the predefined covariate components.

A merging module 190 finally merges 1580 the interpretable covariate model 180 into the interpretable pharmacometric prediction model 150. That is, after the merging step 1580, IPM 150 is again a single model which now also includes components for taking into account covariates when making a prediction on an individual patient's medical state.

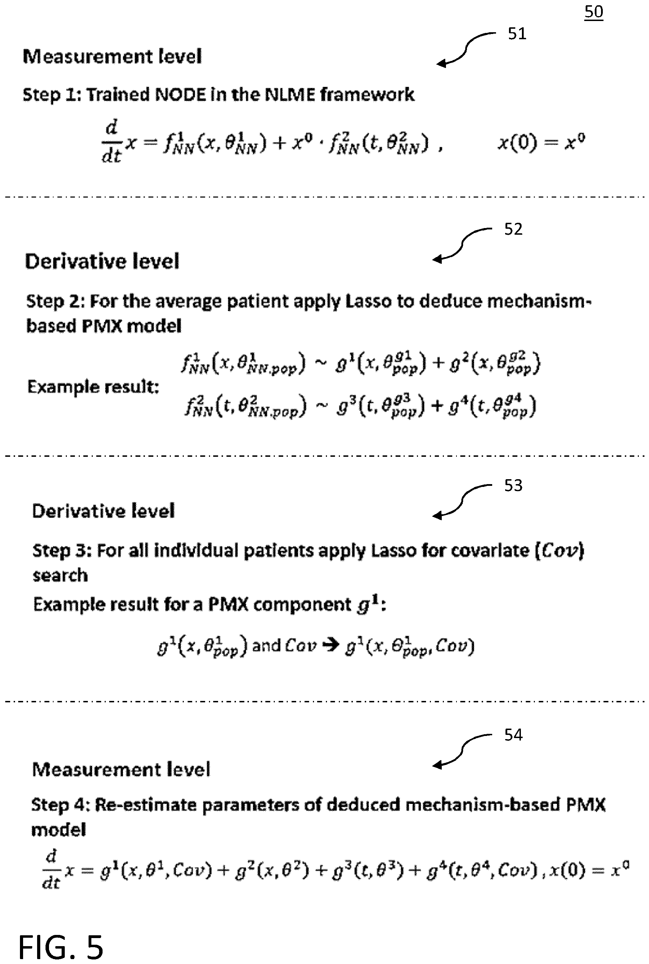

FIG. 5 provides a schematic overview 50 of the above proposed PMX modeling automation workflow including covariate search. It is to be noted that a simplified mathematical representation is applied to not overload the notation of the mathematical terms. In Step 1, on the measurement level 51, the NODE Eq. (2) is trained on the input data, advantageously in the NLME framework. Hence, there are population model parameters as well as the distribution of the random effects. All automation tasks, such as deducing the mechanism-based PMX model (Step 2) and covariate search (Step 3), are performed on the derivative level 52, 53, since the right-hand-side (derivative) was learned by neural networks. Therefore, no solving of differential equations is necessary during the automation task. Finally, the model parameters of the deduced mechanism-based PMX model are reestimated (Step 4) on the measurement level 54, advantageously in the NLME framework.

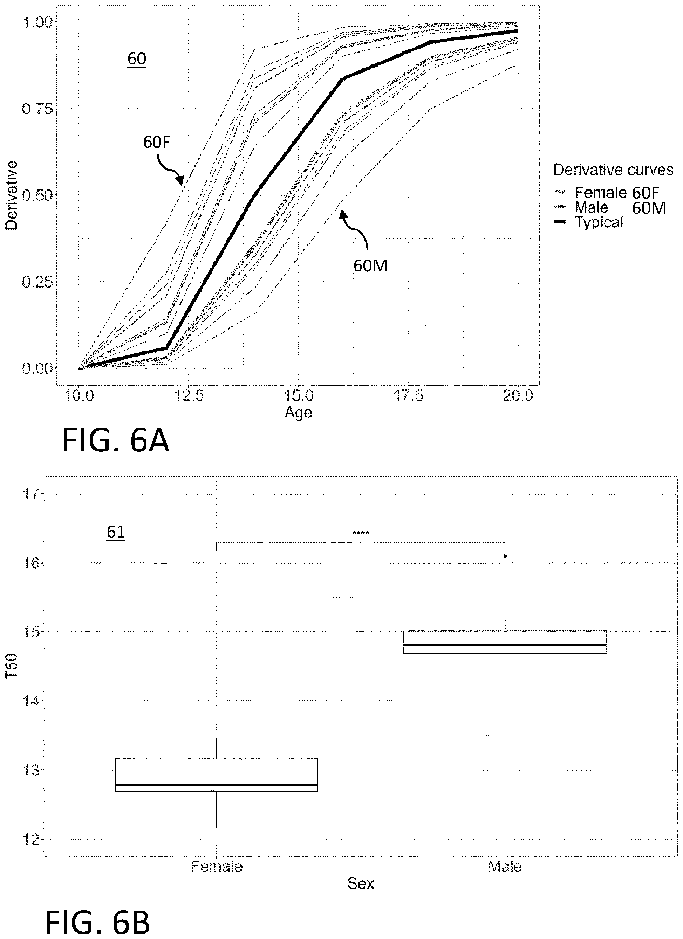

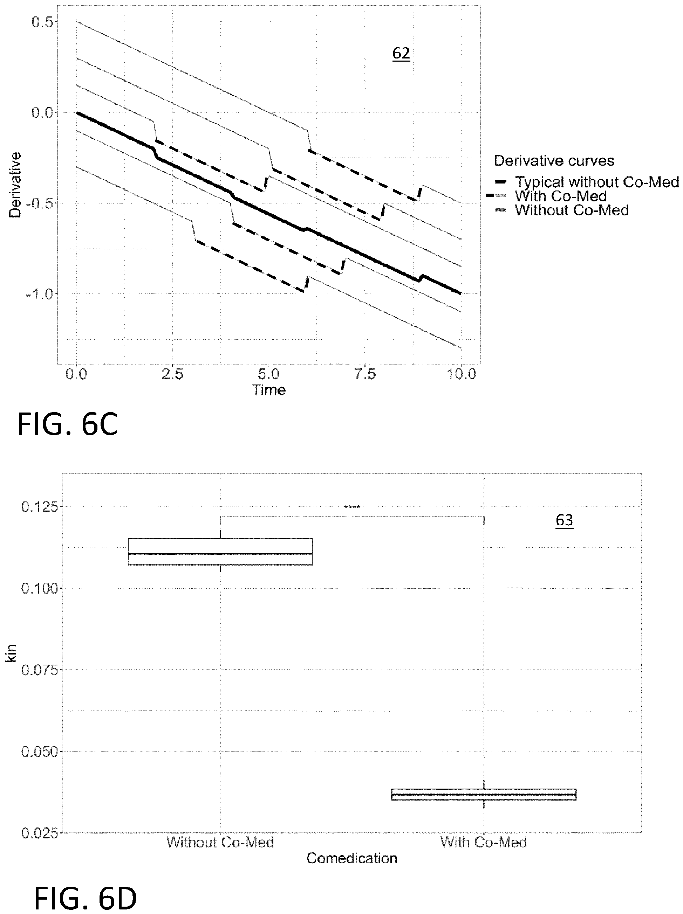

FIGs. 6A, 6B illustrate a first example of a constant covariate analysis where the example data results from PD measurements of hormone levels in adolescents. FIGs. 6C, 6D illustrate a second example of a time varying covariate analysis where the example data results from PK concentration measurements of a first drug while at certain time-points a second drug (Co-Med) was co-administered.

With regard to the constant covariate analysis, the aim is to investigate, whether there are differences in hormone expression development between females and males. Assuming a low-dimensional NODE is fitted, derivative data $dx^t_{NN,pop}$ is generated for typical parameters $\theta_{pop}$ and a sigmoidal component is identified as best-fit component for NAD according to:

$$f^t_{NN}\left(t, \theta_{pop}\right) \sim \frac{t^h}{t^h_{50} + t^h}$$

The following individual output data is generated:

$$dx^t_{NN,i} = f^t_{NN}(t, \theta_i)$$

FIG. 6A illustrates plot 60 of $dx^t_{NN,pop}$ and $dx^t_{NN,i}$ as a derivative-versus-age plot of fitted NNs in NODE with typical and individual estimated parameters. The bold line illustrates the typical parameters. The dark grey lines to the left of the typical parameters are estimated parameters 60F for females and the light grey lines to the right of the typical parameters are estimated parameters 60M for males.

The previously identified best-fit component for NAD is fitted to $dx^t_{NN,i}$ in the NLME framework according to

$$dx^t_{NN,i} \sim \frac{t^{h_i}}{t^{h_i}_{50,i} + t^{h_i}}$$

with random effects on both parameters $t_{50}$ and $h$ in the best-fit component according to

$$t_{50,i} = t_{50,pop} * e^{\eta_{t50,i}}, \qquad \eta_{t50} \sim N(0, \omega^2_{t50})$$

$$h_i = h_{pop} \cdot e^{\eta_{h,i}}, \qquad \eta_h \sim N(0, \omega^2_h)$$

FIG. 6B shows a boxplot 61 of estimated t50s from derivative data stratified by sex and statistical Willcoxen test (**** = p-value < 0.0001) as a statistic test for correlations between $\eta_{t50,i}$ and covariates, indicating a covariate effect of sex on t50.

With regard to the time varying covariate analysis example, the aim is to investigate, whether drug 2 influences the elimination of drug 1. Assuming a low-dimensional NODE is fitted, derivative data $dx^t_{NN,pop}$ is generated for typical parameters $\theta_{pop}$ and a linear component is identified as best-fit component for NAD according to:

$$f^t_{NN}\left(t, \theta_{pop}\right) \sim k_{in} - k_{out} \cdot t$$

The following individual output data is generated:

$$dx^t_{NN,i} = f^t_{NN}(t, \theta_i)$$

Plot 62 of $dx^t_{NN,pop}$ and $dx^t_{NN,i}$ in FIG. 6C is a derivative-versus-time plot of fitted NNs in NODE with typical and individual estimated parameters. The bold curve shows the derivative for typical parameters. The individual parameter derivative curves above and below the typical curve have portions showing the estimates for an individual patient without medication (solid grey line portions) and portions showing estimates with medication (dashed line portions).

The previously identified best-fit component for NAD is fitted to $dx_{NN,i}^t$ in the NLME framework according to

$$dx_{NN,i}^x \sim k_{in,i} - k_{out,i} \cdot x$$

With nested random effects on both parameters $k_{in}$ and $k_{out}$ in the best-fit component according to

$$k_{in,i} = k_{in,pop} * e^{\eta_{kin,i} + \eta_{kin,t(i)}}, \qquad \eta_{kin} \sim N(0, \omega_{kin}^2), \eta_{kin,t(i)} \sim N(0, \omega_{kin,t(i)}^2)$$

$$k_{out,i} = k_{out,pop} * e^{\eta_{kout,i} + \eta_{kout,t(i)}}, \qquad \eta_{kout} \sim N(0, \omega_{kout}^2), \eta_{kout,t(i)} \sim N(0, \omega_{kout,t(i)}^2)$$

where $\eta_{kin,i}$ and $\eta_{kout,i}$ are random effects on subject level, and $\eta_{kin,t(i)}$ and $\eta_{kout,t(i)}$ are nested random effects on time within subject level.

FIG. 6D shows a boxplot 63 of estimated kins from derivative data stratified by comedication and statistical Willcoxen test (*** = p-value < 0.001) as a statistic test for correlations between $\eta_{kin,t(i)}$ and time-varying covariates, indicating a covariate effect of comedication on kin.

The system 100 of FIG. 1 can then be used for predicting at least one medical state parameter MSP1 of a particular patient 201 with characteristics of the special population for which IPM 150 is trained. For this purpose, system 100 is further adapted to implement 1620 IPM 150 in a PMX software. Further, the system obtains 1640 one or more input parameters 211-1 and/or individual model parameters such that:

- one or more input parameters 211-1 for the generated interpretable pharmacometric prediction model 150 are obtained 1641 such that the one or more input parameters, i.e., relevant covariates in the covariate model, relate to the particular patient 201 according to

$$\theta_i = h\big(\theta_{pop}, Cov_i\big)$$

where $\theta_i$ is the individualized model parameters, $\theta_{pop}$ is the previously estimated population parameters, $Cov_i$ are the obtained covariates (input parameters), and $h$ is the covariate model; and/or

- one or more measurements of the medical state parameter of the particular patient $y_i$ 201 are obtained 1642 to further obtain individual model parameters $\theta_i$ with an Empirical Bayesian Estimation (EBE) step according to

$$\hat{\eta}_i = \underset{\eta_i}{\operatorname{argmax}} \, p(\eta_i | y_i; \theta_{pop})$$

$$\theta_i = h(\theta_{pop}, \hat{\eta}_i)$$

where $\theta_{pop}$ is the previously estimated population parameters, $\hat{\eta}_i$ is the estimated individual random effects obtained through the EBE step, and $h$ is the parameter distribution model for the generated interpretable pharmacometric prediction model 150.

System 100 then applies 1660 the interpretable pharmacometric prediction model 150 to said one or more input parameters 211-1 and/or said individual model parameters, and provides 1680 the at least one medical state parameter MSP1 of said particular patient 201. Typically, the medical state parameter MSP1 is then used by a medically trained person for supporting the diagnosis of the patient's medical condition and for taking respective therapeutic actions if appropriate.

In the following, a specific example is discussed in detail which relates to the special population of pediatric patients. The example discloses how to apply NODEs to derive a mechanism-based PMX model for characterizing maturation related serum creatinine dynamics in preterm newborns. Serum creatinine in neonates follows complex dynamics due to maturation processes, most pronounced in the first few weeks of life. The development of a model describing such complex dynamics requires high expertise in pharmacometric modeling. The herein disclosed approach is now utilized to automatically derive a pharmacometric model for serum creatinine in neonates considering maturation processes and covariates.

A steady state between creatinine synthesis and clearance is not reached during the first months of human life because of developmental changes in normal physiology as well as superimposed pathophysiological alterations. Until birth, the fetal renal clearance capacity remains very limited, with the placental circulation acting as a 'physiological hemodialysis circuit', so that fetal serum creatinine values nearly equal maternal ones until (near)term gestational age. From (near)term gestational age onwards, the fetal creatinine values are somewhat higher than the maternal ones, likely because of limitations of the placental clearance capacity and increasing fetal creatinine synthesis (muscle mass related). Once disconnected from the placental circulation at birth, this placental dialysis circuit disappears and will be gradually substituted by an increasing, 'endogenous' renal elimination capacity of the newborn. This phenomenon is mainly driven by a significant increase in renal blood flow from 3% to 25% of the fetal cardiac output, as the neonatal kidney is highly vasoreactive. Based on drug-specific observations in neonates and young infants, renal elimination hereby depends on prenatal (e.g. birth weight or gestational age), and postnatal maturation (e.g. current weight, or postnatal age). In addition, there is some weight loss (-6 to -8 % of the birth weight) in the first days of life, related to water losses, so that, for example, sodium and creatinine may increase due to volume constriction, while an increase in muscle mass will happen once the newborn is in anabolic condition.

Since all these maturational physiological changes are most prominent in early infancy, this is reflected in extensive inter- and intra-individual variability in serum creatinine, resulting in widely dispersed data instead of information that can be interpreted and used at the bedside by health care providers. This pattern results in what is referred to as the 'broken stick' pattern in serum creatinine throughout infancy. Further reflecting on this, a 'hockey' stick is perhaps a more accurate description to focus on the additional variability in the first 6-8 weeks of life. In the neonatal (intensive) care setting, these maturational changes are further modulated by pathophysiological changes like asphyxia, nephrotoxic drugs, or impaired hemodynamics, further increasing this intra- and interindividual variability in serum creatinine values.

Although the relevant covariates are reasonably well identified and quantified, and there is knowledge on the mechanistic understanding, there is a need for supporting a medically trained person with conversions of these phenotypic clouds of serum creatinine in neonates into useful information to guide daily practices in the individual newborn or young infant. This is achieved by the herein disclosed system and methods.

Data for this example was obtained from preterm newborns. Serum creatinine concentrations of the first 6 weeks of life were considered. In addition, clinical data was collected including gestational age (GA), birth weight (BWT), current weight (CWT), sex, mode of delivery (MOD), maternal betamethasone treatment to induce fetal lung maturation, and treatment of the neonate with ibuprofen or inotropic antibiotics. For timepoints without CWT measurement, CWT was interpolated linearly. Patients with no information on GA or MOD were excluded from the analysis.

For evaluation, the dataset was randomly split in a training set, on which the models were developed, and an evaluation set, on which the developed models were evaluated. Neonates were randomly assigned to training (80%) or evaluation set (20%).

To estimate kidney function or glomerular filtration rate (*GFR*), the most measured and readily accessible biomarker in human medicine is serum creatinine (*Scr*). Creatinine is a by-product of the non-enzymatic conversion of creatine to creatinine in the muscle. Creatinine is subsequently cleared from plasma almost exclusively by *GFR* with minimal active secretion by the renal tubules. Assuming steady state, creatinine synthesis (Syn) and clearance are in balance, so that *Scr* values can reliably be used to estimate *GFR*.

$$Scr = \frac{Syn}{GFR} \tag{3}$$

A mixed-effects low-dimensional NODE was utilized to model serum creatinine levels in the population of preterm neonates. The structure of the NODE-based ML model reads

$$\frac{d}{dt}ScrC = f_{NN}^{C}(ScrC) + ScrC_0 \cdot f_{NN}^{t}(t) \qquad ScrC(0) = ScrC_0 \tag{4}$$

where *ScrC* denotes the serum creatinine concentration, $ScrC_0$ serum creatinine concentration at birth, $f_{NN}^{C}$ a serum creatinine concentration dependent NN, $f_{NN}^{t}$ a time dependent NN and *t* time after birth in days. Note that $f_{NN}^{t}$ represents the NN approximating the non-autonomous part of the NODE, i.e., the change in serum creatinine dynamics due to maturation processes in neonates, and $f_{NN}^C$ represents the NN approximating the autonomous part of the NODE, i.e., the overall serum creatinine dynamics. The NN architecture utilized in this example has one hidden layer with 5 neurons and a ReLU activation (cf. https://en.wikipedia.org/wiki/Rectifier_(neural_networks)). The data were modeled in Monolix (Monolix 2023R1, Lixoft SAS, a Simulations Plus company) where model parameters in $f_{NN}^C$ and $f_{NN}^t$, and $ScrC_0$ were estimated, and fitted NODEs were further analyzed in R.

Derivative data was obtained from $f_{NN}^C$ and $f_{NN}^t$ and a LASSO-regression was applied to fit outputs from commonly applied PMX components, i.e., linear functions, exponential functions, and sigmoidal functions, to the derivative data.

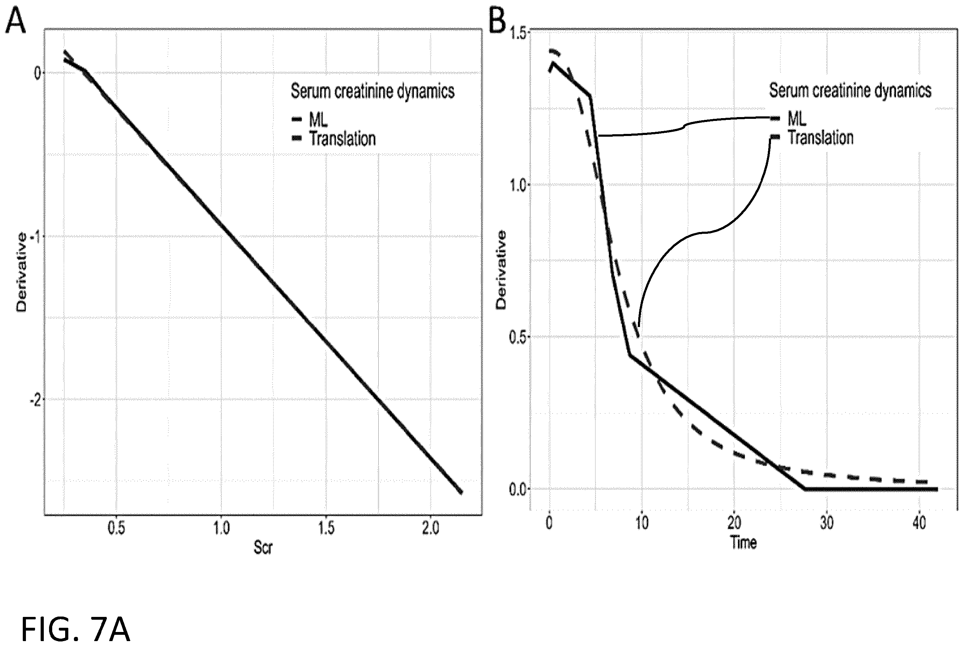

The derivatives obtained from the autonomous, serum creatinine concentration dependent NN, i.e., $f_{NN}^C$, are plotted against serum creatinine in FIG. 7A (section A on left side of the figure). The best-fit component identified through a LASSO-regression was a linear function according to

$$f_{NN}^C(ScrC) \sim k_{in} - k_{out} \cdot ScrC \qquad (8)$$

For consistency with conventional modeling approaches, the y-axis intercept in such a linear function is called $k_{in}$ and represents the production rate of serum creatinine and the slope of the linear function is called $k_{out}$ and represent the elimination rate. The fitted linear function is visualized in FIG. 7A section A. Thereby, the learned dynamics of the NODE based ML approach for the serum creatinine concentration dependent $f_{NN}^C$ (A) and the time-dependent $f_{NN}^t$ (B) is represented by the solid black line (ML) and the corresponding translation to a "mechanism-based" model is represented by the dashed line (Translation).

The derivatives obtained from the non-autonomous, time-dependent NN, i.e., $f_{NN}^t$, are plotted against time in FIG. 7A section B. The best-fit components identified through a LASSO-regression are a sigmoidal Emax and an exponential function. Thus, the following approximation is assumed

$$f_{NN}^t(t) \sim E_{max,in} \cdot \left(1 - \frac{t^h}{t_{50}^h + t^h}\right) + pmax \cdot e^{-k \cdot t} \qquad (9)$$

where $E_{max,in}$ denotes the maximal increased input in unmatured neonates, $h$ the Hillcoefficient, $t_{50}$ the time-point where half the effect is reached, *pmax* the maximal exponential effect and *k* the exponential decay rate.

The resulting interpretable "mechanism-based" model can be written as

$$\frac{d}{dt} ScrC = k_{in} - k_{out} \cdot ScrC + ScrC_0 \cdot E_{max,in} \cdot \left(1 - \frac{t^h}{t_{50}^h + t^h}\right) + pmax \cdot e^{-k \cdot t}, \qquad ScrC(0) = ScrC_0 \qquad (10)$$

The interpretable "mechanism-based" model in Eq. (10) was implemented in Monolix and fitted to the data.

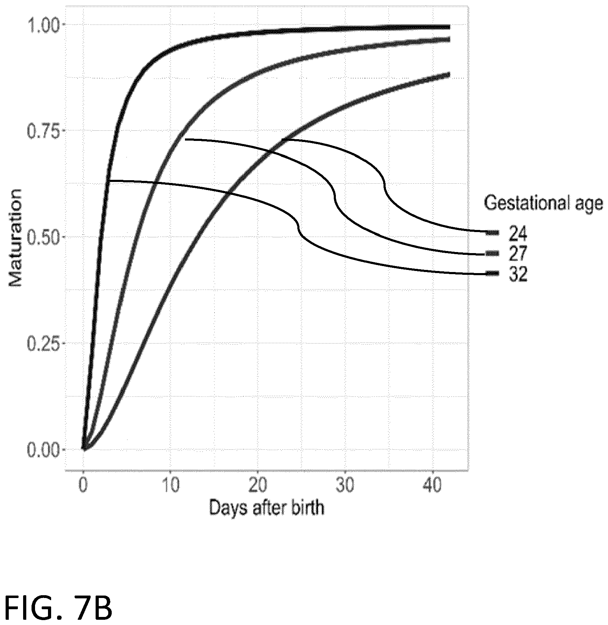

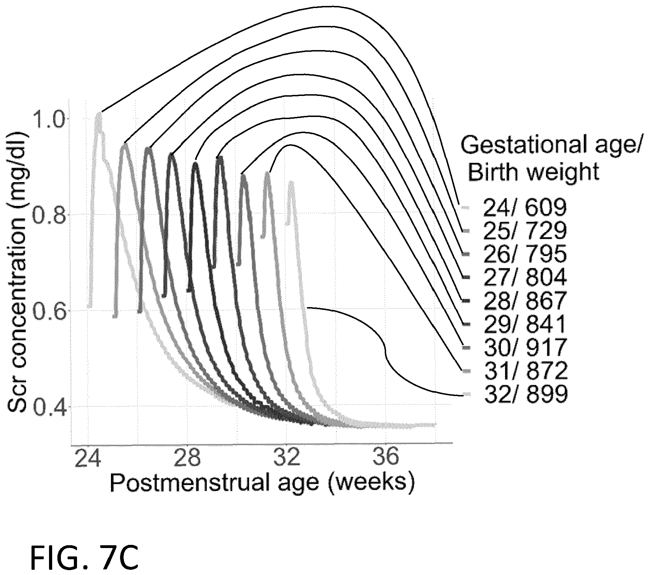

Serum creatinine concentration at birth $ScrC_0$ was estimated at 0.6 mg/dl. GA was identified as covariates for $t_{50}$. This covariate effect is visualized in FIG. 7B. Covariate analysis also showed increasing $ScrC_0$ with increasing GA and BWT. Combined effect of GA on maturation, i.e., $t_{50}$, and of GA and BWT on initial serum creatinine $ScrC$ is visualized in the simulation plot of FIG. 7C showing simulations of serum creatinine concentrations for gestational ages from 24 weeks to 32 weeks with the corresponding median birth weights. The median predictions and the 95% prediction intervals of *Scr* concentrations for neonates with GA of 24, 27, and 32 are generally in accordance with observed *Scr* concentrations and previously published reference values.

The data driven NODE-based ML model in Eq. (4) is capable of describing the complex dynamics of serum creatinine concentrations in preterm neonates well. An interpretable "mechanism-based" model in Eq. (10) could be derived through LASSO-regression from the NODE-based ML model that had reasonable structure for *Scr* concentration dynamics including a maturation process. In the interpretable "mechanism-based" model in Eq. (10), a maturation was proposed where the time to reach maturation was dependent on GA. GA and BWT might be representative for the overall maturity of the neonate at birth and thus influence the initial serum creatinine level and time until maturation process in serum creatinine elimination has completed as maturation process takes longer in neonates with lower GA. This can in part also be explained by the characteristics of the cohort with consequently overrepresentation of growth restricted preterm neonates. Similar behavior can be observed for serum creatinine concentration at birth, as less mature neonates with lower GA tend to have a more pronounced initial increase in serum creatinine concentration after birth.

The presented approach of fitting data first with a data driven NODE-based ML model has the advantage that dynamics of data can efficiently be determined without prior assumptions about the biological processes. Combining the knowledge gained through the dynamics with usually applied functions in pharmacometric models allows to translate such dynamics into an interpretable "mechanism-based" model fully automatically. While the sigmoidal curve in the derivative versus state plot would allow to easily derive the Emax function in the example case, more complex derivative versus state plots can be more challenging to find one or a combination of reasonable pharmacometric functions to describe the learned dynamics visually. Thus, the automatic approach to find the best-fit pharmacometric functions (or components) through a LASSO-regression presents an advantageous approach for translating a machine learning model to an interpretable pharmacometrics model.

The herein disclosed approach can be applied to different medical state parameters of a special population. In the following, two further examples are given in the context of neonatal case studies. The presented examples reflect case studies in neonatology for characterizing and understanding complex physiological changes and maturation processes in newborns: (i) changes of body weight and (ii) changes of the electrolyte serum sodium. For comprehensibility of the herein disclosed AI-PMX reverse modeling approach, derivative-versus-state dependencies are presented as respective plots where derivative data from the NN $dx_{NN}^x$ and output data $dx_i^x$ of the selected mechanistic functions are plotted against $x$, and similarly for the time with $dx_{NN}^t$ and $dx_i^t$ against $t$.

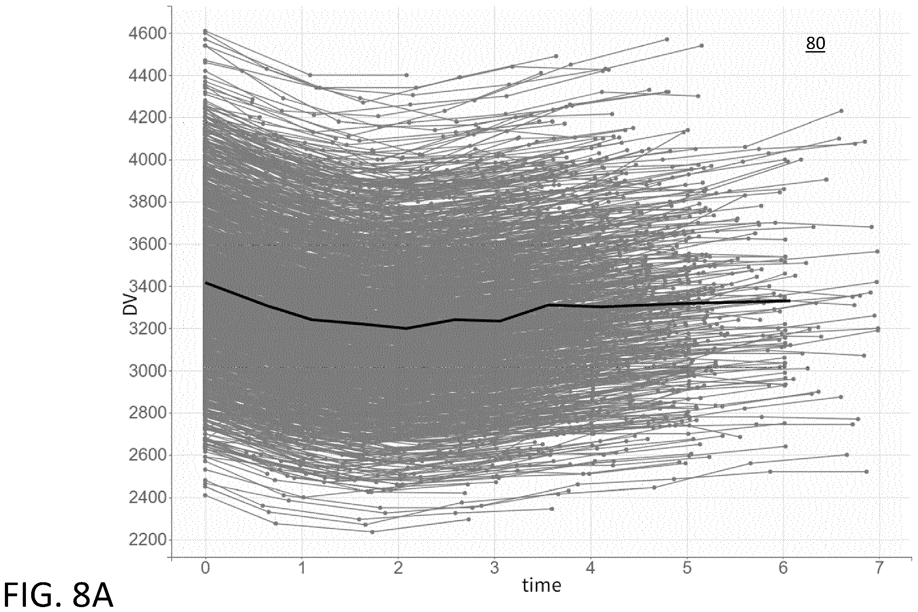

Example (i) relates to physiological body weight changes in newborns during the first week of life. FIG. 8A illustrates a neonatal body weight dataset of weight measurements in over 1000 newborns over a period of 6 days. Conventional, classical models describe body weight changes in newborns having a cut-off in their function to capture the initial decrease followed by an increase in body weight, according to

$$\frac{dW}{dt} = \begin{cases} f_1(W,t) & for\ t < t_1 \\ f_2(W,t) & for\ t \geq t_1 \end{cases}, \qquad W(0) = W_0$$

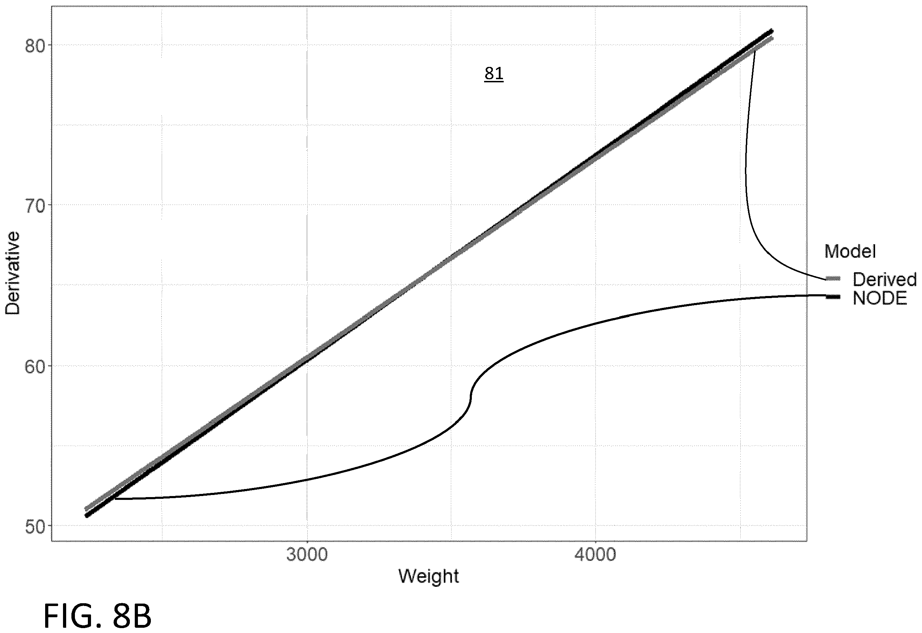

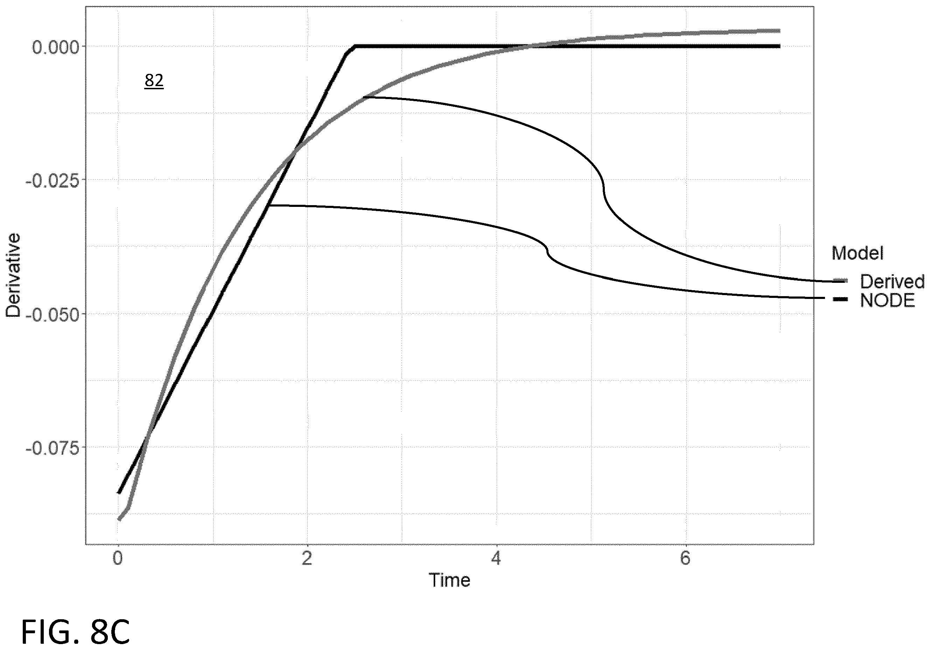

FIGs. 8B, 8C illustrate the result obtained when generating an interpretable PMX model in accordance with the herein described approach. Functions selected with the LASSO-regression from the NN derivative data are:

"Derived" function in plot 81 of FIG. 8B (Derivative-versus-state plot of weight-dependent NN):

$$f_{NN}^x(x, \theta_x) \sim intercept_x + slope_x \cdot x$$

and "Derived" function in plot 82 of FIG. 8C (Derivative-versus-time plot of time-dependent NN):

$$f_{NN}^t(t, \theta_t) \sim intercept_t - Emax_t \cdot \frac{t^h}{t_{50}^h + t^h} - pmax \cdot e^{-k \cdot t}$$

The generated PMX weight model is then implemented according to:

$$\frac{dW}{dt} = intercept_{x,t} + slop_x \cdot W - Emax_t \cdot \frac{t^h}{t_{50}^h + t^h} - pmax \cdot e^{-k \cdot t}, \quad W(0) = W_0$$

The generated "Derived" weight model was fitted in Monolix and model parameters were estimated as shown in table 1:

Table 1 - Model fit with derived neonatal weight model

| Parameter | Estimate | R.S.E. (%) |
|---|---|---|
| $intercept_{x,t}$ | 93.21 | 8.61 |
| $slop_x$ | 0.0054 | 24.9 |
| $Emax_t$ | 64.01 | 16.0 |
| $t_{50}$ | 0.41 | 9.56 |
| $h$ | 0.038 | 22.2 |
| $p_{max}$ | 358.95 | 0.742 |
| $k$ | 0.82 | 1.46 |

Compared to conventional, classical modeling approaches, the NODE-based automatic modeling approach identified functions that have no cut-off in time and thus can reflect maturation more realistic since there is usually no hard cut-off in physiological processes.

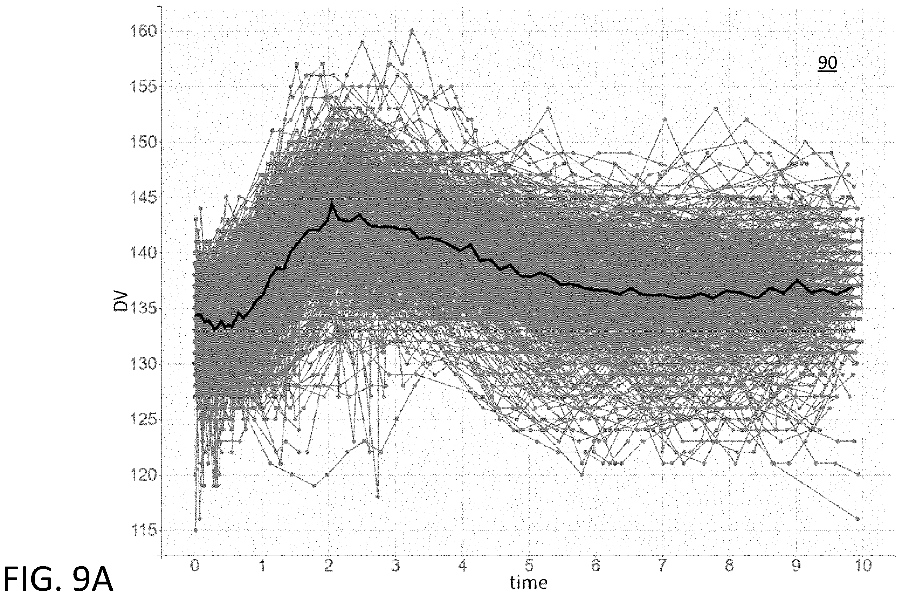

Example (ii) relates to physiological serum sodium changes in newborns. FIG. 9A illustrates neonatal serum sodium dataset generated by sodium measurements in almost 1000 newborns over a period of 10 days. The shape of sodium curves in newborns is very complex with three stages: (a) an initial decrease, (b) an increase with a peak sodium concentration above initial level, (c) a decrease to a stable sodium level. Some classical sodium modeling approaches ignore the initial drop and only focus on the increase and following decrease in sodium levels.

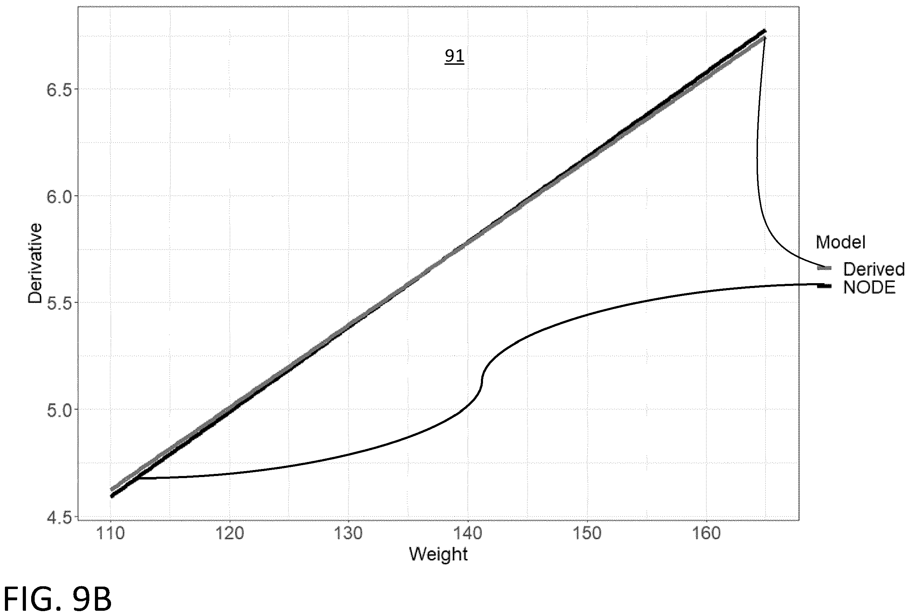

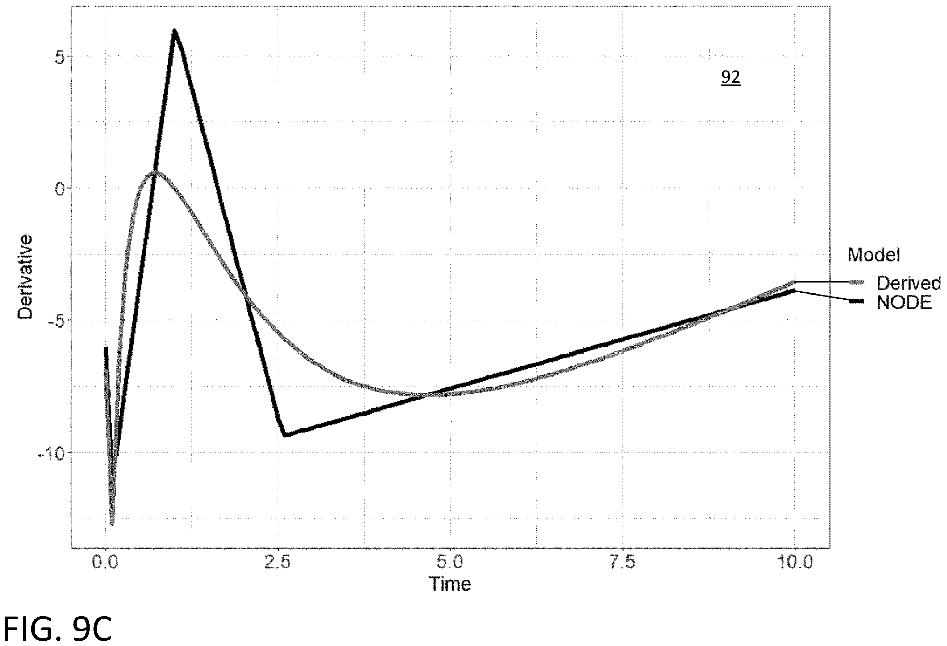

FIGs. 9B, 9C illustrate the result obtained when generating an interpretable PMX model in accordance with the herein described approach. Functions selected with the LASSO-regression from the NN derivative data are:

"Derived" function in plot 91 of FIG. 9B (Derivative-versus-state plot of sodium-dependent NN):

$$f^x_{NN}(x, \theta_x) \sim intercept_x + slope_x \cdot x$$

and "Derived" function in plot 92 of FIG. 9C (Derivative-versus-time plot of time-dependent NN):

$$f^t_{NN}(t, \theta_t) \sim intercept_t - Emax_{1,t} \cdot \frac{t^{h_1}}{t^{h_1}_{1,50} + t^{h_1}} + Emax_{2,t} \cdot \frac{t}{t_{2,50} + t} + pmax \cdot e^{-k \cdot t}$$

The derived sodium model is implemented according to:

$$\frac{dS}{dt} = intercept_{x,t} + slope_x \cdot S - Emax_{1,t} \cdot \frac{t^{h_1}}{t^{h_1}_{1,50} + t^{h_1}} + Emax_{2,t} \cdot \frac{t}{t_{2,50} + t} + pmax \cdot e^{-k \cdot t},$$

$$S(0) = S_0$$

The generated "Derived" weight model was fitted in Monolix and model parameters were estimated as shown in table 2:

Table 2 - Model fit with derived neonatal sodium model

| Parameter | Estimate | R.S.E. (%) |
|---|---|---|
| $intercept_{x,t}$ | -32.23 | 0.177 |
| $slope_x$ | 0.0029 | 5.81 |
| $Emax_{1,t}$ | 49.96 | 0.211 |
| $t_{1,50}$ | 0.072 | 1.63 |
| $h_1$ | 0.092 | 1.63 |

(continued)

| Parameter | Estimate | R.S.E. (%) |
|:---:|:---|:---|
| $Emax_{2,t}$ | 64.29 | 0.108 |
| $t_{2,50}$ | 0.37 | 0.662 |
| $pmax$ | 44.12 | 0.569 |
| $k$ | 0.88 | 0.774 |

Compared to conventional, classical sodium models, the NODE-based automatic modeling approach was able to identify a model that also describes the initial drop in sodium concentrations.

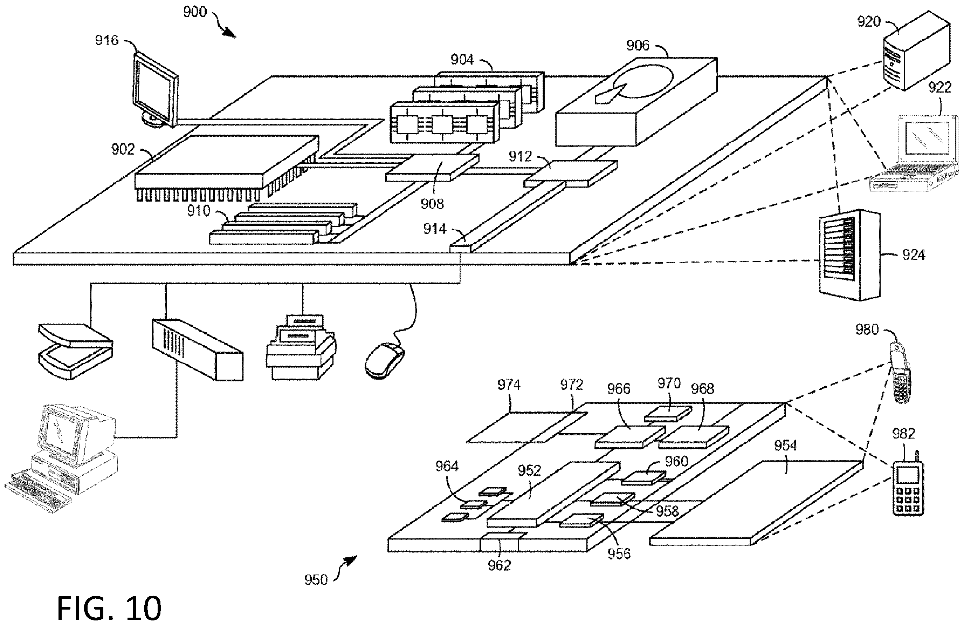

FIG. 10 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, servers, blade servers, mainframes, and other appropriate computers. Generic computer device 900 may correspond to the computer system 100 of FIG. 1 for generating the data driven interpretable pharmacometrics prediction model, as well as for making medical state parameter predictions for a particular patient. Computing device 950 is intended to represent various forms of mobile devices, such as personal digital assistants, cellular telephones, smart phones, and other similar computing devices. For example, computing device 950 may be used as a frontend by a user (e.g., medically trained staff) to interact with the computing device 900, e.g., for visualization of the generated model function, and/or retrieving the predicted medical state parameters. For example, the user may receive realtime monitoring data (e.g., an alarm) about the medical state of a particular intensive care patient while treating another patient. The user can then immediately shift the focus of attention to that particular patient. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low speed interface 912 connecting to low speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high speed interface 908. In other implementations, multiple processors and/or multiple buses may be used, as appropriate, along with multiple memories and types of memory. Also, multiple computing devices 900 may be connected, with each device providing portions of the necessary operations (e.g., as a server bank, a group of blade servers, or a multi-processor system).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid-state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high-speed controller 908 manages bandwidth-intensive operations for the computing device 900, while the low speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a Microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processors. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952 that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or another similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a

middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the scope of the invention.

In addition, the logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

**Claims**

1. Computer-implemented method (1000) for generating a data driven interpretable pharmacometrics prediction model (150) to predict at least one medical state parameter (MSP1) of a patient (201) of a special population, comprising:

   obtaining (1100) a neural ordinary differential equation (110), referred to as low-dimensional NODE, wherein the right-hand side of the NODE has an autonomous part and a non-autonomous part, the NODE comprising at least:

   a first neural network (NN1) trained for approximating the autonomous part describing autonomous dynamics (AD) of at least one particular medical state parameter of special population (200) members, and a second neural network (NN2) trained for approximating the nonautonomous part describing non-autonomous dynamics (NAD) of at least one particular medical state parameter of the special population (200) members, wherein training the neural networks comprises fitting (1120) the NODE (110) to respective data (211) of said special population members;

   computing (1200) derivative data (AD', NAD') generated from the first and second neural networks (NN1, NN2) within minimum and maximum values of observed medical state parameter values (OPR) and time values (OTI); fitting (1300) outputs (O1 to OM) of predefined commonly accepted pharmacometric components (PC1 to PCN) to the derivative data using LASSO regression; and identifying (1400) a particular combination (C1) of best-fit pharmacometric components (PC1, PC2) as the data driven interpretable pharmacometric prediction model (150) which best describes the learned functions of the first and second neural networks (NN1, NN2).

2. The method of claim 1, further comprising:

   fitting (1520) the identified particular combination (C1) of pharmacometric components to derivative data (ADi', NADi') of individual special population members in a non-linear mixed effects modeling framework to determine inter-individual variability in the computed derivative data in form of random effects on model parameters (RE*) in the combination (C1) of best-fit pharmacometric components (PC1, PC2);

fitting (1540) outputs (CO1 to COL) of predefined covariate components (CC1 to CCL) to the random effects (RE*) using LASSO regression; and

identifying (1560) a particular combination (C2) of best-fit covariate components (CO1, CO2) as data driven interpretable covariate model (180) which best describes the influence of covariates on individual dynamics in the special population; and

merging (1580) the interpretable covariate model (180) into the interpretable pharmacometric prediction model (150).

3. The method of claim 1 or 2, further comprising:

implementing (1620) the interpretable pharmacometrics prediction model (150) in a PMX software;

obtaining (1640) at least one of:

one or more input parameters (211-1) for the generated interpretable pharmacometric prediction model (150), wherein the one or more input parameters relate to a particular patient (201) with characteristics of the special population; and

one or more measurements of the medical state parameter of the particular patient (201) with characteristics of the special population to obtain individual model parameters for the generated interpretable pharmacometric prediction model (150) with an Empirical Bayesian Estimation (EBE) step;

applying (1660) the interpretable pharmacometric prediction model (150) to said one or more input parameters (211-1) and/or said individual model parameters; and

providing (1680) the at least one medical state parameter (MSP1) of said particular patient (201).

4. The method of any of the previous claims, wherein the commonly applied pharmacometric components are of any of the following types: linear function, exponential functions, sigmoidal functions, power functions, and hyperbolic functions, and, when dependent on any of claims 2 to 3, the predefined covariate components are of any of said types.

5. The method of any of the previous claims, wherein a selection of the predefined commonly accepted pharmacometric components is received prior to fitting the outputs to the derivative data, wherein the selection comprises components having a visual similarity with corresponding derivative-versus-state dependencies of said derivative data.

6. The method of any of the claims 1 to 4, wherein a respectively trained neural network is used for determining the combinations of the predefined commonly accepted pharmacometric components.

7. The method of any of the previous claims, wherein LASSO regression for fitting (1300) the outputs (O1 to OM) of predefined commonly accepted pharmacometric components (PC1 to PCN) to the derivative data uses L1 penalization of the weight parameters in the linear combination of the pharmacometric components, and sets the weight parameters of unnecessary pharmacometric components to zero.

8. The method of any of the previous claims, wherein the special population is any one of: a population consisting of pediatric members, a population consisting of elderly members, a population consisting of pregnant and breastfeeding women, and a population consisting of members with renal or hepatic impairment.

9. A computer program product for generating a data driven interpretable pharmacometrics prediction model (150) to predict at least one medical state parameter (MSP1) of a patient (201) of a special population, the computer program product comprising computer readable instructions that, when loaded into a memory of a computing device and executed by at least one processor of the computing device, cause the computing device to execute the computer-implemented method according to any of the previous claims.

10. A computer system (100) for generating a data driven interpretable pharmacometrics prediction model (150) to predict at least one medical state parameter (MSP1) of a patient (201) of a special population, comprising:

an interface adapted to obtain a neural ordinary differential equation (110), referred to as low-dimensional NODE, wherein the right-hand side of the NODE has an autonomous part and a non-autonomous part, the NODE comprising at least:

a first neural network (NN1) trained for approximating the autonomous part describing autonomous dynamics (AD) of at least one particular medical state parameter of special population (200) members, and a second neural network (NN2) trained for approximating the nonautonomous part describing non-autonomous dynamics (NAD) of at least one particular medical state parameter of the special population (200) members;

a training module (120) adapted to train the neural networks (NN1, NN2) using a first fitting module (121) adapted to fit the NODE (110) to respective data (211) of said special population members (200);
a derivative data module (130) adapted to compute derivative data (AD', NAD') generated from the neural networks (NN1, NN2) within minimum and maximum values of observed medical state parameter values (OPR) and time values (OTI);
a second fitting module (140) adapted to fit outputs (O1 to OM) of predefined commonly accepted pharmacometric components (PC1 to PCN) to the derivative data (AD', NAD') using a LASSO regression, and further adapted to identify a particular combination (C1) of best-fit pharmacometric components (PC1, PC2) as the data driven interpretable pharmacometric prediction model (150) which best describes the learned functions of the first and second neural networks (NN1, NN2),
and describes the dynamics learned by said neural networks.

11. The system of claim 10, further comprising:

a third fitting module (160) adapted to fit the identified particular combination (C1) of pharmacometric components to derivative data (ADi', NADi') of individual special population members in a non-linear mixed effects modeling framework to determine inter-individual variability in the computed derivative data in form of random effects on model parameters (RE*) in the combination (C1) of best-fit pharmacometric components (PC1, PC2);
a fourth fitting module (170) adapted to fit outputs (CO1 to COL) of predefined covariate components (CC1 to CCL) to the random effects (RE*) using a LASSO regression, and further adapted to identify a particular combination (C2) of best-fit covariate components (CO1, CO2) as data driven interpretable covariate model (180) which best describes the influence of covariates on individual dynamics in the special population; and
a merging module (190) adapted to merge the interpretable covariate model (180) into the interpretable pharmacometric prediction model (150).

12. The system of claim 10 or 11, wherein the system is further adapted to implement the interpretable pharmacometrics prediction model (150) in a PMX software, and to obtain at least one of:

one or more input parameters (211-1) for the generated interpretable pharmacometric prediction model (150), wherein the one or more input parameters relate to a particular patient (201) with characteristics of the special population; and
one or more measurements of the medical state parameter of the particular patient (201) to obtain individual model parameters for the generated interpretable pharmacometric prediction model (150) with an Empirical Bayesian Estimation (EBE) step;

wherein the system is further adapted to apply the interpretable pharmacometric prediction model (150) to said one or more input parameters (211-1) and/or said individual model parameters, and to provide the at least one medical state parameter (MSP1) of said particular patient (201).

13. The system of any of the claims 10 to 12, wherein the commonly applied pharmacometric components are of any of the following types: linear function, exponential functions, sigmoidal functions, power functions, and hyperbolic functions, and, when dependent on any of claims 11 to 12, the predefined covariate components are of any of said types.

14. The system of any of the claims 10 to 13, wherein LASSO regression uses L1 penalization of the weight parameters in the linear combination of the pharmacometric components, and sets the weight parameters of unnecessary pharmacometric components to zero.

15. The system of any of the claims 10 to 14, wherein the special population is any one of: a population consisting of pediatric members, a population consisting of elderly members, a population consisting of pregnant and breastfeeding women, and a population consisting of members with renal or hepatic impairment.

FIG. 1

1500

obtaining nonlinear mixed-effects neural ordinary differential equation NODE comprising first and second neural networks
**1100**

fitting NODE to respective data of special population patients
**1120**

computing derivative data generated from the neural networks within minimum and maximum values of observed medical state parameter values and time values
**1200**

fitting outputs of combinations of predefined commonly accepted pharmacometric components to the derivative data using LASSO regression
**1300**

identifying a particular combination of best-fit pharmacometric components as data driven interpretable prediction model which best describes the learned functions of the first and second neural networks
**1400**

adding interpretable covariate model
**1500**

predicting medical state parameter of particular patient
**1600**

# FIG. 2A

1500

fitting identified particular combination of
pharmacometric components to derivative data of
individual special population patients to determine
random effects on model parameters
1520

fitting outputs of predefined covariate components to
random effects using LASSO regression
1540

identifying particular combination of best-fit covariate
components as data driven interpretable covariate model
which best describes the influence of covariates on
individual dynamics in the special population
1560

merging interpretable covariate model into interpretable
pharmacometric prediction model
1580

# FIG. 2B

1600

implementing interpretable pharmacometrics prediction
model in PMX software
1620

obtaining input
parameter(s) for
generated interpretable
pharmacometric
prediction model relates
to particular patient

1641

obtaining
measurement(s) of
medical state parameter
of particular patient to
obtain individual model
parameters with an
Empirical Bayesian
Estimation (EBE) step
1642

1640

applying interpretable pharmacometric prediction model
to the input parameter(s) and/or individual model
parameters
1660

providing medical state parameter of particular patient
1680

FIG. 2C

FIG. 3

FIG. 4A

FIG. 4B

50

**Measurement level** 51

Step 1: Trained NODE in the NLME framework

$$\frac{d}{dt}x = f_{NN}^1(x, \theta_{NN}^1) + x^0 \cdot f_{NN}^2(t, \theta_{NN}^2) \ , \qquad x(0) = x^0$$

**Derivative level** 52

Step 2: For the average patient apply Lasso to deduce mechanism-based PMX model

Example result:
$$f_{NN}^1(x, \theta_{NN,pop}^1) \sim g^1(x, \theta_{pop}^{g1}) + g^2(x, \theta_{pop}^{g2})$$
$$f_{NN}^2(t, \theta_{NN,pop}^2) \sim g^3(t, \theta_{pop}^{g3}) + g^4(t, \theta_{pop}^{g4})$$

**Derivative level** 53

Step 3: For all individual patients apply Lasso for covariate $(Cov)$ search

Example result for a PMX component $g^1$:

$$g^1(x, \theta_{pop}^1) \text{ and } Cov \rightarrow g^1(x, \theta_{pop}^1, Cov)$$

**Measurement level** 54

Step 4: Re-estimate parameters of deduced mechanism-based PMX model

$$\frac{d}{dt}x = g^1(x, \theta^1, Cov) + g^2(x, \theta^2) + g^3(t, \theta^3) + g^4(t, \theta^4, Cov) , x(0) = x^0$$

# FIG. 5

FIG. 6A

FIG. 6B

**FIG. 6C**

**FIG. 6D**

FIG. 7A

FIG. 7B

FIG. 7C

FIG. 8A

FIG. 8B

EP 4 641 578 A1

FIG. 8C

FIG. 9A

FIG. 9B

FIG. 9C

EP 4 641 578 A1

FIG. 10

Europäisches
Patentamt

European
Patent Office

Office européen
des brevets

# EUROPEAN SEARCH REPORT

**Application Number**

EP 24 17 2640

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/172812 A1 (LU JAMES [US]) 2 June 2022 (2022-06-02) * [0006], [0044], [0063] * | 1-15 | INV. G16H20/10 G16H70/40 |
| A | BRÄM DOMINIC STEFAN ET AL: "Low-dimensional neural ODEs and their application in pharmacokinetics", JOURNAL OF PHARMACOKINETICS AND PHARMACODYNAMICS, vol. 51, no. 2, 14 October 2023 (2023-10-14), pages 123-140, XP93170470, US ISSN: 1567-567X, DOI: 10.1007/s10928-023-09886-4 Retrieved from the Internet: URL:https://link.springer.com/article/10.1007/s10928-023-09886-4/fulltext.html> * par spanning pages 123 and 124, page 125 last par, page 126 first par, page 128 par 2 * | 1-15 | |
| A | Lu James ET AL: "Neural-ODE for pharmacokinetics modeling and its advantage to alternative machine learning models in predicting new dosing regimens", iScience, 30 June 2021 (2021-06-30), pages 1-13, XP93170464, DOI: 10.1016/j.isci Retrieved from the Internet: URL:https://www.ncbi.nlm.nih.gov/pmc/articles/PMC8283337/pdf/main.pdf [retrieved on 2024-09-26] * page 8 last 3 paragraphs * | 1-15 | **TECHNICAL FIELDS SEARCHED (IPC)** <br><br> G16H |

-/--

The present search report has been drawn up for all claims

| Place of search | Date of completion of the search | Examiner |
|---|---|---|
| Munich | 27 September 2024 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 17 2640

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BRÄM DOMINIC STEFAN ET AL: "Low-dimensional neural ODEs and their application in pharmacokinetics", JOURNAL OF PHARMACOKINETICS AND PHARMACODYNAMICS, vol. 51, no. 2, 14 October 2023 (2023-10-14), pages 123-140, XP093170470, US ISSN: 1567-567X, DOI: 10.1007/s10928-023-09886-4 Retrieved from the Internet: URL:https://link.springer.com/article/10.1007/s10928-023-09886-4/fulltext.html> * the whole document * | 1-15 | |

TECHNICAL FIELDS
SEARCHED    (IPC)

The present search report has been drawn up for all claims

| Place of search | Date of completion of the search | Examiner |
|---|---|---|
| Munich | 27 September 2024 | Bankwitz, Robert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 17 2640

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2024

| Patent document cited in search report | Publication date | Patent family member(s) | Publication date |
|---|---|---|---|
| US 2022172812 A1 | 02-06-2022 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHEN R** ; **RUBANOVA Y** ; **BETTENCOURT J** ; **DUVENAUD D**. Neural Ordinary Differential Equations.. *arXiv:180607366.*, 2019
- **LU J** ; **DENG K** ; **ZHANG X** ; **LIU G** ; **GUAN Y**. Neural-ODE for pharmacokinetics modeling and its advantage to alternative machine learning models in predicting new dosing regimens. *iScience*, 2021, vol. 24 (7), 102804
- **BRAM DS** ; **NAHUM U** ; **SCHROPP J** ; **PFISTER M** ; **KOCH G**. Low-dimensional neural ODEs and their application in pharmacokinetics. *Journal of pharmacokinetics and pharmacodynamics*, 2023
- **RIESBECK J.** Systems of linear nonautonomous differential equations. Centre for Mathematical Sciences Lund University, 2020
- **KOCH G** ; **JUSKO WJ** ; **SCHROPP J.** Target mediated drug disposition with drug-drug interaction, Part II: competitive and uncompetitive cases.. *Journal of pharmacokinetics and pharmacodynamics.*, 2017, vol. 44 (1), 27-42